# EUROPEAN PATENT SPECIFICATION

(11) **EP 4 197 318 B1**
(45) Date of publication and mention of the grant of the patent: **19.11.2025**
(21) Application number: 21383157.1
(22) Date of filing: 17.12.2021
(51) Int. Cl.: A01H 4/00

(54) **PHOSPHODIESTERASES INHIBITORS TO PROMOTE IN VITRO PLANT CELL REPROGRAMMING TOWARDS PLANT EMBRYOGENESIS OR MICROCALLUS FORMATION**
PHOSPHODIESTERASEINHIBITOREN ZUR FÖRDERUNG DER IN-VITRO-UMPROGRAMMIERUNG VON PFLANZENZELLEN GEGEN DIE EMBRYOGENESE ODER MICROCALLUS-BILDUNG IN PFLANZEN
INHIBITEURS DE PHOSPHODIESTÉRASES POUR PROMOUVOIR LA REPROGRAMMATION DE CELLULES VÉGÉTALES IN VITRO EN VUE DE L'EMBRYOGENÈSE DE PLANTES OU LA FORMATION DE MICROCALLUS

(43) Date of publication of application: 21.06.2023
(73) Proprietor: Albert-Ludwigs-Universität Freiburg, 79104 Freiburg (DE); ScreenSYS GmbH, 79108 Freiburg i. Br. (DE); Consejo Superior de Investigaciones Científicas (CSIC), 28006 Madrid (ES)
(72) Inventor: Sánchez Testillano, Pilar, Madrid (ES); Martínez Gil, Ana, Madrid (ES); Gil Ayuso-Gontán, Carmen, Madrid (ES); Carneros García, Elena, Madrid (ES); Pérez Pérez, Yolanda, Madrid (ES); Palme, Klaus, Freiburg im Breisgau (DE); Welsch, Ralf, Freiburg (DE); Pandey, Saurabh, Freiburg (DE)
(74) Representative: Pons Ariño, Angel

(56) References cited:
- WO-A1-2019/075295
- WO-A1-2020/214986
- MIRAS-MORENO BEGO�A ET AL: "A metabolomics insight into the Cyclic Nucleotide Monophosphate signaling cascade in tomato under non-stress and salinity conditions", PLANT SCIENCE, ELSEVIER IRELAND LTD, IE, vol. 309, 26 May 2021 (2021-05-26), XP086621846, ISSN: 0168-9452, [retrieved on 20210526], DOI: 10.1016/J.PLANTSCI.2021.110955
- HAYASHIDA YUTA ET AL: "Characterization of the cAMP phosphodiesterase domain in plant adenylyl cyclase/cAMP phosphodiesterase CAPE from the liverwort Marchantia polymorpha", JOURNAL OF PLANT RESEARCH, TOKYO, JP, vol. 135, no. 1, 15 November 2021 (2021-11-15), pages 137 - 144, XP037663562, ISSN: 0918-9440, [retrieved on 20211115], DOI: 10.1007/S10265-021-01359-4
- ANCA MACOVEI ET AL: "The tyrosyl-DNA phosphodiesterase gene family in Medicago truncatula Gaertn.: bioinformatic investigation and expression profiles in response to copper and PEG-mediated stress", PLANTA ; AN INTERNATIONAL JOURNAL OF PLANT BIOLOGY, SPRINGER, BERLIN, DE, vol. 232, no. 2, 11 May 2010 (2010-05-11), pages 393 - 407, XP019848678, ISSN: 1432-2048

## Description

The invention belongs to the field of agrobiotechnology. Particularly, the invention is defined in claims 1 to 11, relating to the use of phosphodiesterases inhibitors to promote *in vitro* plant cell reprogramming towards embryogenesis or microcallus formation and a method thereof, for further plant regeneration.

### BACKGROUND ART

The increasing demand for global food security in the face of a warming climate and a growing population makes that agriculture in the 21st century faces significant pressure worldwide for more efficient and accelerated breeding to generate new crop varieties, with increasing yield and better adapted to adverse environmental conditions. In forestry, improvement programs search for trees better adapted to biotic and abiotic stress conditions.

New gene editing and transformation techniques are able to improve agronomic traits in desired varieties. These techniques require efficient plant regeneration methods after gene edition/transformation. *In vitro* plant embryogenesis and microcallus formation are systems that can be used in plant regeneration, wherein cell reprogramming and proliferation are crucial.

The application of *in vitro* plant embryogenesis, either somatic or micropore embryogenesis, is currently widely exploited to face challenges in this technical field.

Somatic embryogenesis is a type of plant embryogenesis wherein somatic cells are induced to be reprogrammed, further giving rise to vegetative organs or embryos and ultimately to plants. Several methods based on the use of molecules to promote somatic embryogenesis are described in the state of the art.

WO2021103168A1 discloses a method for promoting *Cunninghamia lanceolata* somatic embryogenesis by using salicylic acid.

EP3827665A1 discloses the use of mammal kinase inhibitors, preferably human kinase inhibitors, to promote the induction of *in vitro* plant embryogenesis. Mammal kinase inhibitors showed beneficial effects in both crop and forest plants in *in vitro* systems of microspore and somatic embryogenesis.

Other type of plant embryogenesis is microspore embryogenesis, wherein microspores are reprogrammed towards an embryogenic pathway to produce a haploid embryo, which can further produce, plants.

Several methods based on the use of molecules in microspore cultures are described in the state of the art.

Inhibition of histone deacetylase (HDAC) activity to promote microspore embryogenesis is disclosed in the document ES2753213T3, which describes a method for producing a haploid plant embryo comprising growing or seeding haploid plant material in the presence of a hydroxamic acid compound or a cyclic tetrapeptide having HDACi activity.

US2002151057 A1 describes methods for generating double-haploid maize plants, comprising a step of culturing the microspores with a specific culture medium, which includes at least one cytokinin and at least one auxin.

The exposure of *Equisetum arvense* and *Hippeastrum hybridum* microspores to different chemical signals such as neurotransmitters acetylcholine, dopamine, and serotonin, their agonists and antagonists, Na⁺, K⁺, and Ca²⁺ channel blockers, as well as forskolin and theophylline, has also been studied (Roshchina, V.V., Biol Bull Russ Acad Sci 33, 332-338 (2006)). Both types of microspores exposed to neurotransmitters, their agonists, forskolin, and theophylline demonstrated growth activation, while neurotransmitter antagonists and ion channel blockers inhibited this process.

Microcallus formation, a process that is based in the induction of previous cell reprogramming and proliferation, has also plant regeneration application, due to the undifferentiated cells that are part of the microcallus.

In Damm B. and Willmitzer L., Mol Gen Genet., 213:15-20 (1988), a protocol for obtaining regenerated fertile plants from mesophyll protoplasts of Arabidopsis thaliana was described. In the protocol, protoplasts were cultured in different media that produces microcalluses, calluses and ultimately rooted shoots.

In Jeong Y. et al., Plant Methods., 17(1):21 (2021), *in vitro* culture conditions for initial cell division, microcallus formation, and *de novo* shoot and root regeneration were disclosed.

However, even though the application of plant embryogenesis, either somatic or microspore embryogenesis, and microcallus formation are currently widely exploited, these processes are still highly, or even completely inefficient in many plant species of economic interest. The yield of these processes has several bottlenecks, being one of the major problems the low proportion of cells that are reprogrammed.

Therefore, new strategies are needed to improve *in vitro* plant cell reprogramming towards plant embryogenesis and microcallus formation in species of economic interest.

### DESCRIPTION OF THE INVENTION

The present invention discloses a novel use of phosphodiesterase (PDE) inhibitor/s, particularly mammalian PDE inhibitor/s, to promote *in vitro* plant cell reprogramming towards plant embryogenesis or microcallus formation for further plant regeneration, and a method thereof. In the present invention, treatments with these inhibitors have been successfully applied in different *in vitro* protocols, in liquid and solid media, in a crop, a forest and an herbaceous plant.

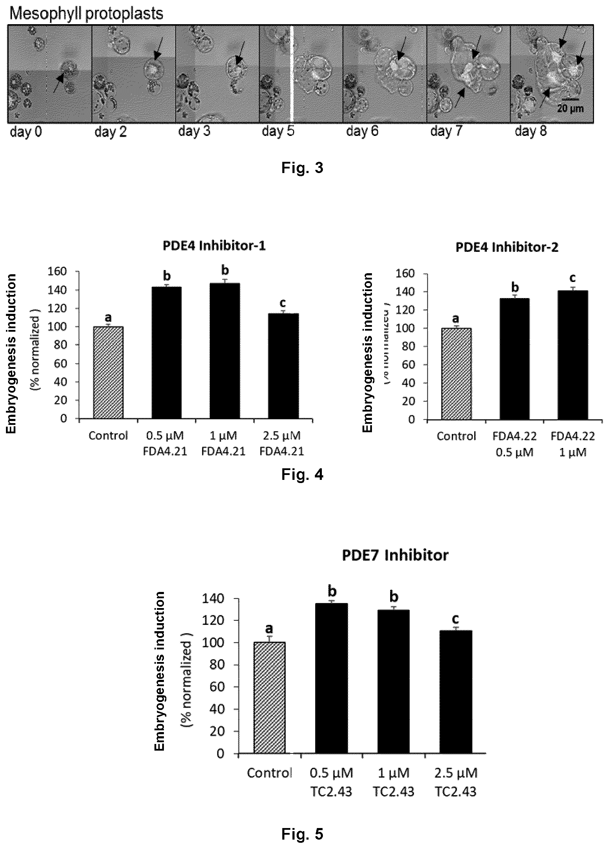

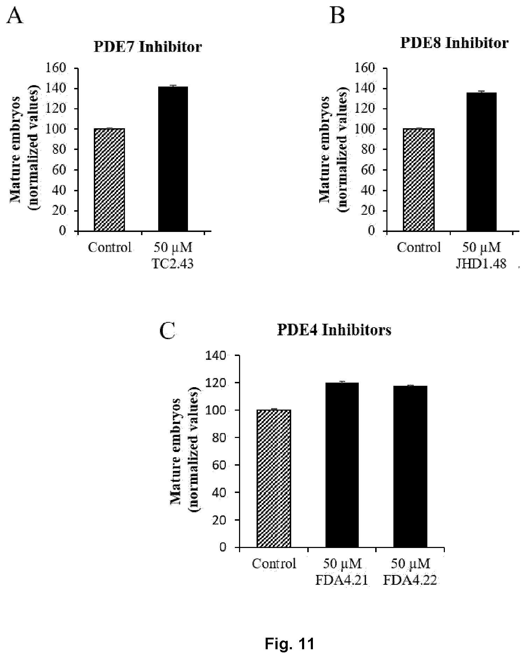

Inventors have observed that the application of mammalian PDE inhibitors, in isolated plant material cultures induces a significant increase of embryogenesis initiation rate in a crop and a forest specie, particularly the inventors have demonstrated enhancing somatic embryogenesis (Figure 11) and microspore embryogenesis (Figures 4 to 7).

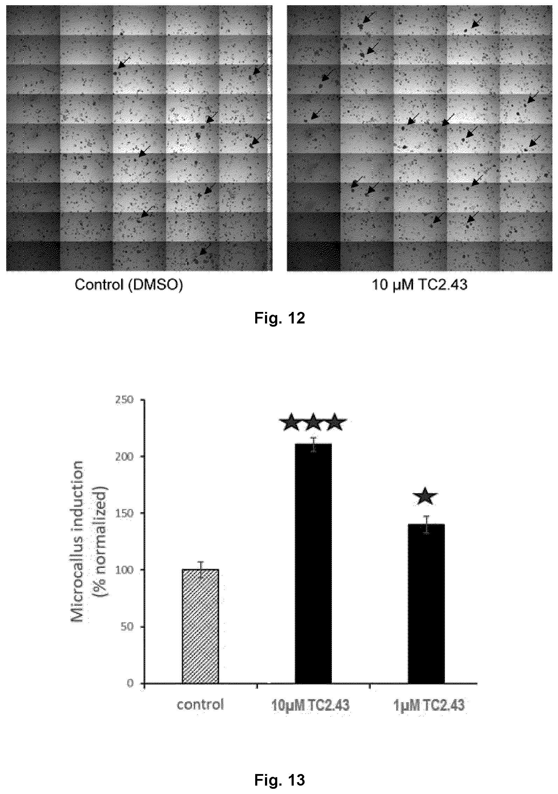

Moreover, presence of some of these mammalian PDE inhibitors in protoplasts culture wherein the protoplast was isolated from *Arabidopsis thaliana,* particularly isolated from mesophyll and root tissues, increased the rate of microcallus formation which is the requirement for efficient regeneration into plants (Figures 13 to 15), with a tissue independent effect of the inhibitors.

Thus, a first aspect of the present invention relates to the use of at least one phosphodiesterase inhibitor (PDE inhibitor), preferably mammalian PDE inhibitor, hereinafter the "inhibitor of the invention", to promote *in vitro* plant cell reprogramming towards plant embryogenesis or microcallus formation, hereinafter the "use of the invention", wherein the PDE inhibitor is selected from the list consisting of:
(i) 3-(2,6-Difluorophenyl)-2-methylthio-4-oxo-3,4-dihydroquinazoline,
(ii)(R)-N-(3-chlorobenzyl)-1-((2-(2-fluorophenyl)-5-methyloxazol-4-yl)methyl)piperidin-3-carboxamide,
(iii) 2-(2-Bromophenyl)-4,5-bis(4-methoxyphenyl)-1H-imidazole,
(iv)3-(cyclopropylmethoxy)-N-(3,5-dichloropyridin-4-yl)-4-(difluoromethoxy)benzamide,
(v)4-(3-cyclopentyloxy-4-methoxyphenyl)pyrrolidin-2-one
and any combination thereof.

The term "phosphodiesterases" or "PDEs" comprise a group of enzymes that degrade the phosphodiester bond in the second messenger molecules cyclic adenosine monophosphate (cAMP) and cyclic guanosine monophosphate (cGMP). They regulate the localization, duration and amplitude of cyclic nucleotide signalling within subcellular domains.

The PDE inhibitor may inhibits specific types of phosphodiesterases, particularly mammalian PDEs. The term "mammalian" as used herein refers to any animal classified as a mammal including, without limitation to, human beings, horses, cows, dogs, cats, rats, mice and primates. The PDE superfamily of enzymes is classified into 11 families in mammals, namely PDE1 to PDE11, i.e., PDE1, PDE2, PDE3, PDE4, PDE5, PDE6, PDE7, PDE8, PDE9, PDE10 and PDE11.

In a preferred embodiment of the invention the PDE is a mammalian PDE.

In other preferred embodiment of the invention the mammalian PDE is selected from the list consisting of: PDE4, PDE7, PDE8, PDE10 and any combination thereof.

The term "phosphodiesterase inhibitor" or "PDE inhibitor" refers to any molecule which is able to completely or partially inhibiting phosphodiesterase activity. In the present invention, "phosphodiesterase activity", refers to the catalytic activity which comprises the hydrolysis of cAMP and/or cGMP. Thus, inhibition of PDE activity may increase intracellular levels of cyclic adenosine monophosphate (cAMP) and/or cyclic guanosine monophosphate (cGMP) in cells.

It is routine practice for a person skilled in the art the identification of PDE inhibitors through methodologies and techniques known in the state of the art, such as, but without limitation to, PDE activity assays, western blotting, ligand-based virtual screening, structure-function studies, advanced proteomics, X-ray crystallographic analysis of PDEs and PDE-inhibitor complexes, or other strategies based on the examination of the allosteric interactions between the catalytic and regulatory domains of PDEs and specific PDEs targeting, as well as high-throughput screening (HTS) of library compounds based on PDE activity/inhibition determination.

Furthermore, as a person skilled in the art knows, PDE inhibition determination can be carried out through methodologies known in the state of the art, that may be based on radiometric inhibition assays, such as, but without limitation to, scintillation proximity assay from Perkin Elmer (Waltham, US) (TRKQ7090), fluorescence intensity measurements after PDE enzyme incubation with PDE inhibitor/s, such as, but without limitation to fluorescence polarization (FP) assay protocol, colorimetric assays, such as, but without limitation to, enzyme systems with reagent for phosphate detection that allow the subsequent PDE inhibition detection, or luminescent assays, among others.

Several PDE inhibitors are disclosed in the state of the art (Maurice, et al., Nat Rev Drug Discov., 13(4):290-314 (2014)), as well as small molecules with specific PDE family inhibition: Thioxoquinazoline derivatives as PDE7 inhibitors are disclosed in Castaño et al., ChemMedChem., 4(5):866-76 (2009); piperidine derivatives as PDE8 inhibitors are disclosed in ES2696516A1; imidazole derivatives as PDE10 inhibitors are disclosed in Garcia et al., Future Med Chem., 9(8):731-748 (2017). PDE 4 inhibitors are disclosed in Li H, et al., Front Pharmacol., 9:1048 (2018).

In the present invention, the PDE inhibitor is selected from the list consisting of:
(i) 3-(2,6-Difluorophenyl)-2-methylthio-4-oxo-3,4-dihydroquinazoline (or TC2.43, terms used interchangeably in the present invention), with the following chemical structure: 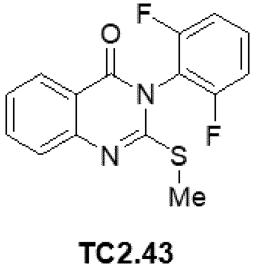
(ii) (R)-N-(3-chlorobenzyl)-1-((2-(2-fluorophenyl)-5-methyloxazol-4-yl)methyl) piperidin-3-carboxamide (or JHD1.48, terms used interchangeably in the present invention), with the following chemical structure: 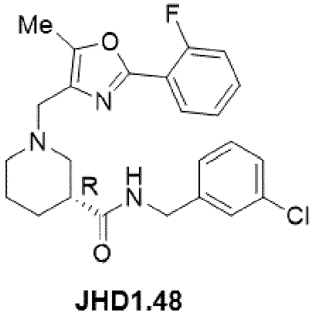
(iii) 2-(2-Bromophenyl)-4,5-bis(4-methoxyphenyl)-1H-imidazole (or AGF4.17, terms used interchangeably in the present invention), with the following chemical structure: 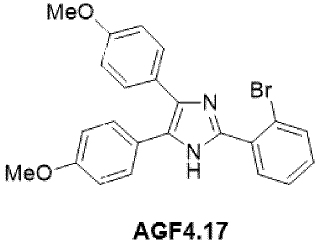
(iv) 3-(cyclopropylmethoxy)-N-(3,5-dichloropyridin-4-yl)-4-(difluoromethoxy) benzamide (or FDA4.21 or Roflumilast, terms used interchangeably in the present invention), with the following chemical structure: 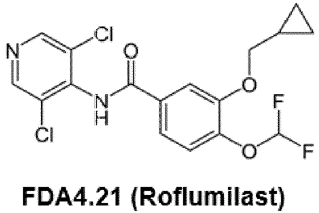
(v) 4-(3-cyclopentyloxy-4-methoxyphenyl)pyrrolidin-2-one (or FDA4.22 or Rolipram, terms used interchangeably in the present invention), with the following chemical structure: 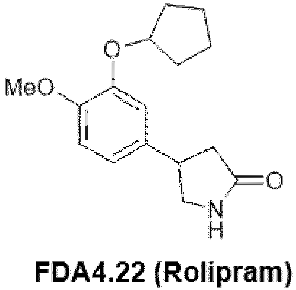
and any combination thereof.

In a particular embodiment, the PDE inhibitor is a PDE4 inhibitor, preferably is 3-(cyclopropylmethoxy)-N-(3,5-dichloropyridin-4-yl)-4-(difluoromethoxy)benzamide (FDA4.21) and/or 4-(3-cyclopentyloxy-4-methoxyphenyl)pyrrolidin-2-one (FDA4.22).

In another particular embodiment, the PDE inhibitor is a PDE7 inhibitor, preferably is 3-(2,6-Difluorophenyl)-2-methylthio-4-oxo-3,4-dihydroquinazoline (TC2.43).

In another particular embodiment, the PDE inhibitor is a PDE8 inhibitor, preferably is (R)-N-(3-chlorobenzyl)-1-((2-(2-fluorophenyl)-5-methyloxazol-4-yl)methyl)piperidin-3-carboxamide (JHD1.48).

In another particular embodiment, the PDE inhibitor is a PDE10 inhibitor, preferably is 2-(2-Bromophenyl)-4,5-bis(4-methoxyphenyl)-1H-imidazole (AGF4.17).

As mentioned above, inventors of the present invention have demonstrated that the application of PDE inhibitor/s, preferably mammalian PDE inhibitor/s, promotes *in vitro* plant cell reprogramming towards plant embryogenesis or microcallus formation

The term "promote", used in the present invention along with "plant cell reprogramming", "plant embryogenesis", "somatic embryogenesis", "microspore embryogenesis" and/or "microcallus formation" refers to the fostering or induction of this process/es.

The term *"in vitro",* used in the present invention along with "plant cell reprogramming", "plant embryogenesis", "somatic embryogenesis", "microspore embryogenesis" and/or microcallus formation refers to this/these process/es in a cell culture or in an isolated biological sample, outside the plant organism.

As used herein, the term "plant cell reprogramming" refers to a process for altering the natural state of the plant cell such that changes its developmental program (followed in planta) towards a new pathway which makes the cell capable of being further developed into an embryo, embryo-like structure, or a microcallus. Plant cell reprogramming can include inducing pluripotency or totipotency in or de-differentiation of the plant cell or enhancing the level of pluripotency or totipotency or de-differentiation that has been induced by an PDE inhibitor.

The expression "towards plant embryogenesis or microcallus formation", as used herein, relates to the plant embryogenesis or microcallus formation induction caused by previous plant cell reprogramming and proliferation. In the present invention, PDE inhibitors promote plant cell reprogramming and proliferation towards plant embryogenesis or microcallus formation for further plant regeneration.

"Plant embryogenesis", as used herein, refers to the process by which plant embryos or proembryos form and develop. As it is known by a person skilled in the art, plant embryogenesis can be induced *in vitro* under appropriate conditions, such as chemical stress conditions, that cause the induction of previous cell reprogramming and proliferation, and subsequent plant embryogenesis. It is routine practice for a person skilled in the art to assess the *in vitro* plant embryogenesis, induction efficiency through the quantification of plant proembryos and/or embryos formed.

The term "embryo", as used herein, refers to an organized structure capable of germinating *in vitro* to produce a plantlet or a plant. As it is known by a person skilled in the art, embryos can be identified through standard techniques known in the state of the art, such as, but without limitation to, stereo microscopy.

The term "proembryo", as used herein, refers to a morphological state, which is the first sign of embryogenesis initiation. As it is known by a person skilled in the art, proembryos can be identified through standard techniques known in the state of the art, such as, but without limitation to, microscopy.

"Microcallus formation", as used herein refers to the process by which microcalli, proliferating mass of undifferentiated cells, are obtained from plant material. This process can be induced *in vitro* under appropriate conditions, such as chemical stress conditions, that cause the induction of previous cell reprogramming and proliferation and subsequent microcallus formation. It is routine practice for a person skilled in the art to assess the *in vitro* microcallus formation efficiency or rate, through the quantification of proliferating cells with shape differences associated with proliferating microcalli. This quantification can be performed, without limitation, by microscopy nuclei visualization based on the expression of nuclear localized histone 2B fused with yellow fluorescent protein (H2B-YFP).

In a preferred embodiment, inhibitor of the invention is used in isolated protoplasts, to promote plant cell reprogramming towards microcallus formation. Methods for protoplast isolation are known in the state of the art. In a particular embodiment, protoplasts are isolated from plant hypocotyl, cotyledon leaf and/or any combination thereof.

As it is known by a person skilled in the art, *in vitro* microspore embryogenesis and somatic embryogenesis are kinds of *in vitro* plant embryogenesis, which are widely used in plant breeding and agrobiotechnology. In the present invention, fostering of *in vitro* plant cell reprogramming towards plant embryogenesis, either microspore embryogenesis or somatic embryogenesis, is induced by the use of the inhibitors of the invention.

Thus, a further embodiment of the present invention provides the use of the inhibitor/s of the invention to promote *in vitro* microspore embryogenesis or somatic embryogenesis.

The term "microspore embryogenesis" as used herein refers to the activation of the process by which microspores give rise to the development of embryos, through a series of cellular divisions under appropriate conditions. As it is used herein, "microspores" refers to immature male gametophytes or precursors of pollen grains (haploid cells) of a plant at all stages of its *in vitro* growth. Microspore embryogenesis may be induced by exposing microspores to abiotic or chemical stress during *in vitro* culture. Stress conditions reprograms microspores and may develop into haploid or doubled-haploid embryos, and eventually into plants.

The term "somatic embryogenesis" as used herein refers to the development of an embryo, derived from somatic cells (cells others than gametes) and protoplasts, under appropriate conditions. In this embryo-forming process, somatic cells acquire embryogenic competence under stress conditions that induce cell reprogramming, which further give rise to vegetative organs or embryos and ultimately to plants.

Examples of somatic cells, without limitation, are cells derived from hypocotyl, from petiole, from leaf, from root, from cotyledon, from anther, from anther filament, from ovule, from petal or from immature zygotic embryo.

Inventors of the present invention have applied the inhibitor/s of the invention at different concentrations, enhancing proliferation and plant cell reprogramming towards plant embryogenesis or microcallus formation. Thus, in a particular embodiment the inhibitor of the invention concentration is from 0.10 to 100 µM.

It is routine practice for a person skilled in the art that the inhibitor/s of the invention concentrations required for promoting *in vitro* plant cell reprogramming towards plant embryogenesis or microcallus formation depend on the culture material and method used. For instance, solid media involve less diffusion and availability of compounds to cells in comparison with liquid media.

For this reason, in some embodiments, inhibitor concentrations are higher when used in solid media than in liquid media. Thus, in a particular embodiment, the inhibitor of the invention concentration in solid media is from 20 to 80 µM. Preferably, the inhibitor of the invention concentration is from 30 to 70 µM, more preferably is from 40 to 60 µM.

In a more preferred embodiment, the inhibitor of the invention concentration is selected from the list consisting of: 40 µM, 41 µM, 42 µM, 43 µM, 44 µM, 45 µM, 46 µM, 47 µM, 48 µM, 49 µM, 50 µM, 51 µM, 52 µM, 53 µM, 54 µM, 55 µM, 56 µM, 57 µM, 58 µM, 59 µM and 60 µM. More preferably, the inhibitor of the invention concentration is 50 µM.

In an even more preferred embodiment, when the PDE inhibitor/s of the invention is 3-(2,6-Difluorophenyl)-2-methylthio-4-oxo-3,4-dihydroquinazoline (TC2.43), 3-(cyclopropylmethoxy)-N-(3,5-dichloropyridin-4-yl)-4-(difluoromethoxy)benzamide (FDA4.21), 4-(3-cyclopentyloxy-4-methoxyphenyl) pyrrolidin-2-one (FDA4.22) and/or (R)-N-(3-chlorobenzyl)-1-((2-(2-fluorophenyl)-5-methyloxazol-4-yl)methyl)piperidin-3-carboxamide (JHD1.48), and it is used in solid media promoting *in vitro* plant cell reprogramming towards plant embryogenesis, preferably, the PDE inhibitor concentration is 50 µM.

In some embodiments, inhibitor concentrations are lower when used in liquid media. Thus, in another particular embodiment, the inhibitor of the invention concentration in liquid media is from 0.10 to 19 µM.

In a preferred embodiment, when the inhibitor of the invention is used in liquid media and promotes *in vitro* plant cell reprogramming towards plant embryogenesis the inhibitor concentration is from 0.10 to 2.50 µM. Preferably, the inhibitor concentration is from 0.20 to 2 µM. In a still more preferred embodiment, the inhibitor of the invention concentration is selected from the list consisting of: 0.20 µM, 0.25 µM , 0.30 µM, 0.35 µM, 0.40 µM, 0.45 µM, 0.50 µM, 0.55 µM, 0.60 µM, 0.65 µM, 0.70 µM, 0.75 µM, 0.80 µM, 0.85 µM, 0.90 µM, 0.95 µM, 1 µM, 1.05 µM, 1.10 µM, 1.15 µM, 1.20 µM, 1.25 µM, 1.30 µM, 1.35 µM, 1.40 µM, 1.45 µM, 1.50 µM, 1.55 µM, 1.60 µM, 1.65 µM, 1.70 µM, 1.75 µM, 1.80 µM, 1.85 µM, 1.90 µM, 1.95 µM, and 2 µM. More preferably, the inhibitor concentration is 0.5 µM or 1 µM.

In a particular embodiment, when the PDE inhibitor/s of the invention is 3-(cyclopropylmethoxy)-N-(3,5-dichloropyridin-4-yl)-4-(difluoromethoxy)benzamide (FDA4.21) and/or 4-(3-cyclopentyloxy-4-methoxyphenyl) pyrrolidin-2-one (FDA4.22), and it is used in liquid media promoting *in vitro* plant cell reprogramming towards plant embryogenesis, preferably, the PDE inhibitor concentration is 0.50 µM or 1 µM, more preferably 1 µM.

In another particular embodiment, when the PDE inhibitor/s of the invention is 3-(2,6-Difluorophenyl)-2-methylthio-4-oxo-3,4-dihydroquinazoline (TC2.43) and/or 2-(2-Bromophenyl)-4,5-bis(4-methoxyphenyl)-1H-imidazole (AGF4.17), and it is used in liquid media promoting *in vitro* plant cell reprogramming towards plant embryogenesis, preferably, the PDE inhibitor concentration is 0.50 µM or 1 µM, more preferably is 0.50 µM.

In another particular embodiment, when the PDE inhibitor/s of the invention is (R)-N-(3-chlorobenzyl)-1-((2-(2-fluorophenyl)-5-methyloxazol-4-yl)methyl)piperidin-3-carboxamide (JHD1.48), and it is used in liquid media promoting *in vitro* plant cell reprogramming towards plant embryogenesis, preferably, the PDE inhibitor concentration is 0.50 µM.

In another particular embodiment, when the PDE inhibitor of the invention is used in liquid media and promotes *in vitro* plant cell reprogramming towards microcallus formation, the PDE inhibitor concentration is from 1 to 19 µM. Preferably, the PDE inhibitor concentration is from 5 to 15 µM. In another more particular embodiment, the PDE inhibitor concentration is 5 µM, 6 µM, 7 µM, 8 µM, 9 µM, 10 µM, 11 µM, 12 µM, 13 µM, 14 µM or 15 µM. More preferably, the PDE inhibitor of the invention concentration is 10 µM.

In an even more preferred embodiment, when the PDE inhibitor/s of the invention is 3-(2,6-Difluorophenyl)-2-methylthio-4-oxo-3,4-dihydroquinazoline (TC2.43), (R)-N-(3-chlorobenzyl)-1-((2-(2-fluorophenyl)-5-methyloxazol-4-yl)methyl)piperidin-3-carboxamide (JHD1.48) and/or 2-(2-Bromophenyl)-4,5-bis(4-methoxyphenyl)-1H-imidazole (AGF4.17), and it is used in liquid media promoting *in vitro* plant cell reprogramming towards microcallus formation, the PDE inhibitor concentration is 10 µM.

As it has been previously described, the PDE inhibitors of the invention have been successfully applied in a forest, a crop and an herbaceous plant, fostering plant cell reprogramming towards plant embryogenesis or microcallus formation.

Thus, a particular embodiment of the present invention provides the use of the PDE inhibitor/s of the invention to promote *in* vitro plant cell reprogramming towards plant embryogenesis or microcallus formation, wherein the plant is a crop plant, a forest plant or an herbaceous plant.

A particular embodiment of the present invention provides the use of the PDE inhibitor/s of the invention to promote *in* vitro plant cell reprogramming towards plant embryogenesis, preferably towards somatic embryogenesis or microspore embryogenesis, wherein the plant is a crop or a forest plant.

In a preferred embodiment, the crop plant as used herein is selected from the list consisting of: *Medicago spp., Prunus spp., Angelica spp., Pimpinella spp., Ceratonia siliqua, Malus spp., Areca spp, Arracacia spp, Maranta spp., Cynara spp., Daucus carota, Anacardium occidentale, Asparagus spp., Persea spp., Pearl spp., Pennisetum spp., Vigna spp., Musa spp., Sechium edule, Jatropha spp., Cocos nucifera, Hordeum spp., Apium graveolens, Cyclamen spp., Atalantia spp. Anethum graveoles, Vigna subterranea, Laurus spp., Phaseolus spp. Ocumum spp., Cinnamomum verum, Paulinia cupana, Areca spp., Annona reticulate, Piper spp., Acacia spp., Rubus spp. Vaccinium spp. Bertholletia excelsa, Sesamum indicum, Artocarpus spp., Vicia spp, Fagopyrum esculentum, Carum carvi, Elettaria cardamomum, Ricinus communis, Castanea sativa, Cicer spp. Cichorium spp, Eugenia aromatica, Syzygium aromaticum, Trifolium spp. Erythroxypum spp., Cola spp., Brassica spp., Valerianella locusta, Gossypium spp., Lepidium sativum, Cucumis spp., Ficus carica, Corylus spp., Furcraea macrophylla, Linum spp., Geranium spp., Zingiber spp., Panax spp., Ribes spp., Vitis vinifera, Lygeum spartum, Dactylis spp., Arachis hypogaea, Corylus avellana, Cannabis sativa, Crotalaria juncea, Lawsonia inermis, Armoracia rusticana, Indigofera tinctoria, Jasminum spp., Helianthus spp., Actinidia deliciosa, Lavandula spp., Citrus spp., Cymbopogon citratus, Lens culinaris, Lespedeza spp., Lactuca spp., Litchi chinensis, Eriobotrya japonica, Lupinus spp., Macadamia spp., Zea mays, Mangifera spp., Secale spp, Setaria italica, Echinochloa esculenta, Pennisetum americanum, Panicum miliaceum, Mentha spp., Morus spp., Sinapis spp., Avena spp., Elaeis guineensis, Abelmoschus esculentus, Hibiscus esculentus, Olea spp, Allium spp., Papaver spp., Borassus flabellifer, Elaeis guineensis, Pastinaca sativa, Pisum sativum, Pyrus communis, Carya illinoensis, Capsicum spp., Cajanus cajan, Ananas comosus, Pistacia vera, Punica granatum, Solamum spp., Ipomoea spp. Cucurbita spp., Chrysanthemum spp., Aspidosperma spp., Cydonia oblonga, Cinchona spp., Chenopodium quinoa, Raphanus sativus, Rubus spp., Agrostis spp., Rheum spp., Oryza spp., Rose spp., Hevea brasiliensis, Lolium spp. Crocus savitus, Vitellaria paradoxa, Butyrospermum parkii, Agave spp.,Glycine spp., Triticum spp., Spinacia oleracea, Fragaria spp., Beta spp., Sorghum spp., Thymus spp., Timothy spp., Phleum pratense, Phleum alpinum, Saccharum officinarum, Nicotiana spp., Bixa spp, Solanum spp., Lotus spp., Triticale (Hybrid of Triticum aestivum and Secale cereale), Curcuma spp., Vanilla planifolia, Juglans spp., Citrullus lanatus, Dioscorea spp., Ilex paraguariensis, Pennisetum glaucum, Setaria italic, Eleusine coracana, Panicum virgatum, Echinochloa frumentacea, Paspalum scrobiculatum, Digitaria exilis, Milium effusum, Phalaris canariensis,*and *Coix lacryma-jobi.* Where so applicable the crop plants species here listed include all different sub-species, varieties and cultivars existent, including, without limitation, regional, seasonal and agricultural varieties.

In a more preferred embodiment, the crop plant is selected from the list consisting of: *Hordeum spp., Zea mays, Secale spp, Setaria italica, Panicum miliaceum, Avena spp., Oryza spp., Triticum spp, Sorghum spp., Triticale (Hybrid of Triticum aestivum and Secale cereale), Pennisetum glaucum, Eleusine coracana, Phalaris canariensis, Cynara spp., Daucus carota, Piper spp, Trifolium spp, Brassica spp, Lactuca spp, Mentha spp, Allium spp., Pisum sativum, Capsicum spp, Solamum spp, Cucurbita spp, Chenopodium quinoa, Rubus spp, Spinacia oleracea, Beta spp, Solanum spp., Helianthus spp., Gossypium spp, Arachis hypogaea, Cannabis sativa, Saccharum officinarum, Linum spp., Glycine spp., Nicotiana spp, Medicago spp.,* and *Agrostis spp.*

More preferably, the crop plant belongs to the *Brassica spp.,* even more preferably to *Brassica napus sp.*

In another preferred embodiment, the forest plant is selected from the list consisting of: *Araucaria spp., Cryptomeria japonica, Cupressus spp, Juniperus spp., Sequoia sempervirens, Sequoiadendron giganteum, Thuja spp, Abies spp., Cedrus spp, Larix spp, Picea spp., Pinus spp., Pseudotsuga spp, Taxus spp., Ginkgo biloba, Acer spp., Anacardium occidentale, Mangifera spp, Pistacia spp, Cocos nucifera, Phoenix spp, Betula spp., Corylus spp, Paulownia tomentosa, Adansonia spp, Capparis spp, Sambucus spp., Carica papaya, Euonymus spp, Hevea brasiliensis, Manihot spp., Acacia spp., Robinia spp, Castanea spp, Fagus spp, Quercus spp., Carya spp., Juglans spp., Cinnamomum spp., Laurus spp, Persea spp., Swietenia spp., Artocarpus spp., Ficus spp., Morus spp., Myrtus communis, Psidium spp., Nothofagus spp., Fraxinus spp., Olea europaea, Platanus spp, Dendrocalamus asper, Malus spp., Photinia spp, Photinia* × *fraser, Prunus spp., Pyrus spp., Coffea spp., Citrus spp., Populus spp., Salix spp., Solanum erianthum; Theobroma cacao, Camellia spp., Tilia spp., Ulmus spp.,* Tamarillo, (*Cyphomandra betacea (Cav.) (Sendtn.); Solanum betaceum Cav.),* Indian olive (*Elaeocarpus robustus L.);* bottle palm (*Hyophorbe lagenicaulis*)*,* Indian rosewood (*Dalbergia sissoo*)*,* canela petrea (*Ocotea catharinensis* Mez.), Sandalwood (*Santalum album*), *Echinacea purpurea* L., longan (*Dimocarpus longan* Lour.), (*Aspidosperma polyneuron* Mull.Arg), rattan (*Calamus* spp.), jojoba (*Simmondsia chiensis), (Aegle marmelos* L.), black cohosh *(Actaea racemosa* L.), *Gomortega keule, Cyclamen* spp., Hybrid Aspen (*Populus tremuloides x Populus tremula*)*,* Oil palm (*Elaeis guineensis* Jacq.), *Passiflora* spp., Açaí palm (*Euterpe oleracea* Mart.), tree-fern (*Cyathea delgadii* Sternb.), *Eucalyptus* spp., Hybrid Larch (*Larix x eurolepis* Henry) and neem (*Azadirachta indica*)*.* Where so applicable the forest plants species here listed include all different sub-species, varieties and cultivars existent, including, without limitation, regional, seasonal and agricultural varieties.

In a more preferred embodiment, the forest plant is selected from the list consisting of: *Araucaria spp., Cupressus spp, Juniperus spp., Abies spp., Cedrus spp, Larix spp, Picea spp., Pinus spp., Pseudotsuga spp, Taxus spp., Ginkgo biloba, Acer spp., Anacardium occidentale, Mangifera spp, Pistacia spp, Cocos nucifera, Phoenix spp, Betula spp., Corylus spp, Carica papaya, Hevea brasiliensis, Acacia spp., Robinia spp, Castanea spp, Fagus spp, Quercus spp., Cinnamomum spp., Laurus spp, Persea spp., Morus spp., Psidium spp., Fraxinus spp., Olea europaea, Platanus spp, Malus spp., Prunus spp., Pyrus spp., Coffea spp., Citrus spp., Populus spp., Salix spp., Theobroma cacao, Camellia spp., Ulmus spp., Tamarillo, (Cyphomandra betacea (Cav.) (Sendtn.)* and *Eucalyptus spp.*

More preferably, the forest plant belongs to the *Quercus spp.,* even more preferably to *Quercus suber sp.*

A further embodiment of the present invention provides the use of the PDE inhibitor/s of the invention to promote *in* vitro plant cell reprogramming towards microcallus formation, wherein the plant is an herbaceous plant.

In a preferred embodiment, the herbaceous plant as used herein is selected from the list consisting of:
*Arabidopsis spp., Campanula latifolia spp., Geranium psilostemon spp., Iris latifolia spp., Rodgersia aesculifolia spp., Artemisia ludoviciana 'Silver King' spp., Kniphofia hybrids spp., Lychnis coronaria spp., Stachys byzantina spp., Alchemilla mollis spp., Astilbe x arendsii spp., Hakonechloa macra 'Aureola' spp., Nepeta x faassenii spp., Allium christophii spp., Campanula persicifolia spp., Goniolimon tataricum spp., Tanacetum parthenium 'Aureum' spp., Allium cernuum spp., Adiantum venustum spp., Deschampsia cespitosa spp., Ophiopogon planiscapus spp., Athyrium filix-femina spp., Kirengeshoma palmata spp., Equisetum hyemale spp., Thymus serpyllum spp., Agastache foeniculum spp., Erigeron karvinskianus spp., Oenothera biennis spp., Bidens aurea spp., Helianthus annuus spp., Bergenia crassifolia spp., Iberis umbellata spp., Liriope muscari spp., Pontederia cordata spp., Asclepias incarnates spp., Helianthemum nummularium spp.,and Rhodiola rosea spp.*

Preferably, the herbaceous plant belongs to *Arabidopsis spp.,* more preferably to *Arabidopsis thaliana sp.*

Application of the PDE inhibitors of the invention in plant cultures has demonstrated a significant increase of embryogenesis rate and microcallus formation, following plant cell reprogramming. Inventors of the present invention have developed a method to increase the induction of, or to promote, *in vitro* plant cell reprogramming towards plant embryogenesis or microcallus formation using the PDE inhibitor/s of the invention.

Thus, a second aspect of the present invention relates to a method of promoting *in vitro* plant cell reprogramming towards plant embryogenesis or microcallus formation, hereinafter the "method of the invention", comprising:
a) Culturing an isolated plant material with at least one PDE inhibitor, wherein the PDE inhibitor is selected from the list consisting of:
   (i) 3-(2,6-Difluorophenyl)-2-methylthio-4-oxo-3,4-dihydroquinazoline,
   (ii)(R)-N-(3-chlorobenzyl)-1-((2-(2-fluorophenyl)-5-methyloxazol-4-yl)methyl)piperidin-3-carboxamide,
   (iii) 2-(2-Bromophenyl)-4,5-bis(4-methoxyphenyl)-1H-imidazole,
   (iv)3-(cyclopropylmethoxy)-N-(3,5-dichloropyridin-4-yl)-4-(difluoromethoxy)benzamide,
   (v) 4-(3-cyclopentyloxy-4-methoxyphenyl)pyrrolidin-2-one
and any combination thereof.

The terms "plant cell reprogramming", "plant embryogenesis", "microcallus formation" and "PDE inhibitor" have already been described in a previous aspect of the present invention and applies equally to this aspect as well as all its preferred embodiments.

As used herein, the term "culturing" refers to the process of culture a part of an organism, in the present invention an isolated plant material, outside of its natural environment under particular conditions.

The term "isolated plant material", as used in the present invention, refers to isolated plant cells or isolated plant parts, such as plant organs and tissues. Examples of isolated plant material are, without limitation to, plant protoplasts, plant calli, plant clumps, acorns, seeds, leaves, stems, hypocotyls, cotyledons, pollen grains, microspores, roots, root tips, anthers, anther filaments, ovules, petals, flowers, seedlings, embryos and bolls.

A particular embodiment of the present invention provides the method of the invention wherein the isolated plant material are isolated plant cells, preferably an isolated microspore or an isolated protoplast.

In a preferred embodiment of the method of the invention, to promote *in vitro* plant cell reprogramming towards microcallus formation, the isolated plant material is a protoplast. Preferably, the protoplast is isolated from plant hypocotyl, cotyledon leaf and/or any combination thereof.

Another particular embodiment provides the method of the invention wherein the isolated plant material is a plant part, preferably a plant organ or a plant tissue.

Another particular embodiment provides the method of the invention wherein the isolated plant material is an acorn or an immature zygotic embryo.

As disclosed above, the method of the invention comprises culturing an isolated plant material with at least one PDE inhibitor, preferably mammalian PDE inhibitor. Thus, in a preferred embodiment of the method of the invention the PDE inhibitor is a mammalian PDE inhibitor.

In other more preferred embodiment of the method of the invention, the mammalian PDE is selected from the list consisting of PDE4, PDE7, PDE8, PDE10 and any combination thereof.

In the present invention, the PDE inhibitor is selected from the list consisting of:
(i) 3-(2,6-Difluorophenyl)-2-methylthio-4-oxo-3,4-dihydroquinazoline (TC2.43),
(ii)(R)-N-(3-chlorobenzyl)-1-((2-(2-fluorophenyl)-5-methyloxazol-4-yl)methyl) piperidin-3-carboxamide (JHD1.48),
(iii) 2-(2-Bromophenyl)-4,5-bis(4-methoxyphenyl)-1H-imidazole (AGF4.17),
(iv)3-(cyclopropylmethoxy)-N-(3,5-dichloropyridin-4-yl)-4-(difluoromethoxy)benzamide (FDA4.21),
(v) 4-(3-cyclopentyloxy-4-methoxyphenyl)pyrrolidin-2-one (FDA4.22)
and any combination thereof.

In the present invention, treatments with these inhibitors have demonstrated enhancing somatic and microspore embryogenesis. As it has been described above, *in vitro* microspore embryogenesis and somatic embryogenesis are kinds of *in vitro* plant embryogenesis, which are widely used in plant breeding and agrobiotechnology.

Thus, a further embodiment of the present invention provides the method of the invention wherein the plant embryogenesis is somatic embryogenesis or microspore embryogenesis. The terms "somatic embryogenesis" and "microspore embryogenesis" have already been described in a previous aspect of the present invention and applies equally to this aspect as well as all its preferred embodiments.

As it is known by a person skilled in the art, different culture media may be used depending on the isolated plant material. Generally, the type of medium depends on whether the cells to be cultured are isolated (liquid medium cultures) or whether they are grouped into tissues or organs (solid medium cultures).

Thus, a further embodiment of the present invention provides the method of the invention, wherein the isolated plant material is cultured in a solid medium or in a liquid medium.

A particular embodiment provides the method of the invention wherein the isolated plant material is cultured in a liquid medium when the isolated plant material are isolated plant cells, preferably an isolated microspore or an isolated protoplast.

Another particular embodiment provides the method of the invention wherein the isolated plant material is cultured in a solid medium when the isolated plant material is a plant part, a plant organ or a plant tissue, preferably an acorn or an immature zygotic embryo.

The term "culture media" as used herein is intended to indicate any material either solid or liquid in which plant cells, tissues, organs and whole plants may grow. Additives may be provided to the cells in the form of media, and environmental conditions controlled. There are many types of plant culture media comprised of mixtures of mineral salts containing essential oligoelements plus various additives like amino acids, sugars, growth regulators and vitamins which must therefore be added to the culture medium to allow development of (pro)embryo, explant and/or plant growth. Examples of plant culture media are, without limitation, Chu (N6) medium (Duchefa, Sigma-Aldrich), Clc/lpomoea CP medium (Duchefa), CLC/lpomoea ep medium (Duchefa), DKV/Junglans medum (Duchefa, Sigma-Aldrich), Erikson medium (Duchefa), Gamborg B5 medium (Duchefa, Sigma-Aldrich), Gresshoff and Doy medium (Duchefa), Lindemann orchid medium (Duchefa), NLN medium (Duchefa), Nitsch medium (Duchefa), Woody plant medium (Duchefa, Sigma-Aldrich), Linsmaier and Skoog medium (Duchefa), Litvay medium (Duchefa), Quorin and Lepoivre medium (Duchefa), Rugini olive medium (Duchefa), Schenk and Hildebrant medium (Duchefa, Sigma-Aldrich), White's medium (Duchefa, Sigma-Aldrich), Westvaco WV5 medium (Duchefa), Murashige and Skoog medium (Duchefa, Sigma-Aldrich), Murashige and Skoog medium with B5 vitamins (Duchefa), Murashige and Skoog medium with Nitsch vitamins (Duchefa), Murashige and Skoog medium van der Salm (Duchefa), Hoagland's n°2 basal salt mixture (Sigma-Aldrich), Sommer macronutrients + MS micronutrients and vitamins (Testillano et al. 2018, Plant Cell Culture Protocols, eds. V.M. Loyola-Vargas & N. Ochoa-Alejo. Springer and Bussines Media. pp. 247-256), cultivation medium composed of macro and micro elements as described in Kao KN and Michayluk MR, Planta 126:105-110 (1975) or KBP medium (Kumlehn et al. 2006, Plant Biotechnology Journal, 4: 251-261 (2006).

As a person skilled in the art knows, the culture medium can comprise other compounds and elements that may be necessary, such as, plant hormones.

Plant hormones (also known as phytohormones) are organic substances that regulate plant growth and development. Plant hormones can be classified into different types, including without limitation to, auxins (including synthethic auxins), gibberellins, abscisic acid, cytokinins, salicylic acid, ethylene, jasmonates, brassinosteroids, and peptides.

Examples of plant hormones include, without limitation to, BAP (6-benzylaminopurine), NAA (1-naphtalene acetic acid), brassinolide, 2,4-dichlorophenoxyacetic acid (2,4-D), thidiazurone, indoleacetic acid (IAA), gibberellic acid, 6-furfurylaminopurine, ethylene, phenylacetic acid or 4-chloro-indoleacetic acid.

In a particular embodiment of the method of the invention, the culture medium, preferably a liquid culture medium, further comprises a plant hormone selected from the list consisting of BAP (6-benzylaminopurine), NAA (1-naphtalene acetic acid), brassinolide, 2,4-dichlorophenoxyacetic acid (2,4-D), thidiazurone, indoleacetic acid (IAA), gibberellic acid, 6-furfurylaminopurine, ethylene, phenylacetic acid, 4-chloro-indoleacetic acid and any combination thereof.

In another particular embodiment of the method of the invention, the culture medium, preferably a liquid culture medium, further comprises at least one auxin, preferably the auxin is 2,4-D, and/or at least one cytokinin, preferably, the cytokinin is thidiazurone

In another particular embodiment of the method of the invention, the plant hormone concentration is from 1 to 3 µM. Preferably, the plant hormone concentration is 2 µM.

In another more particular embodiment of the method of the invention, to promote *in vitro* plant cell reprogramming towards microcallus formation, 2,4-D concentration is 2 µM.

In another more particular embodiment of the method of the invention, to promote *in vitro* plant cell reprogramming towards microcallus formation, 2,4-D concentration is 2 µM and thidiazurone concentration is 1 µM.

The duration of culturing the isolated plant material with the PDE inhibitor/s of the invention may depends on the culture material, method used, and type of isolated plant material. For instance, solid media involve less diffusion and isolated plant material is cultured with the inhibitor of the invention a longer time than in liquid media.

Thus, in a particular embodiment of the method of the invention, the isolated plant material is cultured with the inhibitor of the invention for 1 to 20 days.

In other particular embodiment of the method of the invention, when the isolated plant material is cultured in a solid medium with the inhibitor of the invention, it is cultured for 10 to 20 days, preferably 15 days.

In another particular embodiment of the method of the invention, when the isolated plant material is cultured in a liquid medium with the inhibitor of the invention, it is cultured for 1 to 6 days, preferably 2 to 4 days.

In a preferred embodiment, when the isolated plant material is a microspore, it is cultured for 4 days.

In another preferred embodiment, when the isolated plant material is a protoplast, it is cultured for 2 days.

As it is known by a person skilled in the art, the temperature of culturing the isolated plant material with the PDE inhibitor/s of the invention may depends on the culture material, method used, and type of isolated plant material.

Thus, in a particular embodiment of the method of the invention, the isolated plant material is cultured with the inhibitor of the invention at a temperature from 20°C to 35°C.

In other particular embodiment of the method of the invention, the isolated plant material is cultured with the inhibitor of the invention at a temperature selected of the list consisting of: 21 °C, 22 °C, 23 °C, 24 °C, 25 °C, 26 °C, 27 °C, 28 °C, 29 °C, 30 °C, 31 °C and 32 °C, preferably 22°C, 25°C or 32°C.

As it is known by a person skilled in the art, depending on the isolated plant material and/or type of embryogenesis, other steps may be needed in order to prepare the isolated plant material before the application of a stress condition or chemical treatment, such as the inhibitors of the invention.

Thus, in a particular embodiment of the method of the invention, to promote cell reprogramming towards somatic embryogenesis in a solid medium, the method further comprises the following step previous to the treatment with the PDE inhibitor/s of the invention:
a') culturing the isolated plant material in a culture medium which comprises at least one plant hormone, obtaining proembryogenic masses.

As it has been described, this pre-treatment step a') is a previous step to the treatment with the inhibitor/s of the invention. In the present invention, the culture medium of the step a'), hereinafter the "induction medium", comprises at least one plant hormone. Then, the proembryogenic masses obtained are transferred and cultured with the inhibitor/s of the invention (step a)), wherein the proembryogenic masses proliferate and form embryos.

The term "proembryogenic masses", as used herein, refers to cellular aggregates composed of embryogenic cells, whose proliferation is a main proembryogenic stage feature.

In a particular embodiment of the method of the invention, the plant hormone is selected of the list consisting of: BAP (6-benzylaminopurine), NAA (1-naphtalene acetic acid), brassinolide, 2,4-dichlorophenoxyacetic acid (2,4-D), thidiazurone, indoleacetic acid (IAA), gibberellic acid, 6-furfurylaminopurine, ethylene, phenylacetic acid, 4-chloro-indoleacetic acid and any combination thereof.

In other particular embodiment of the method of the invention, the plant hormone is an auxin. Preferably, the auxin is 2,4-dichlorophenoxyacetic acid (2,4-D).

The molecule "2,4-dichlorophenoxyacetic acid (2,4-D)" is a synthetic auxin, which is widely used as a growth regulator in *in vitro* plant cultures, such as somatic embryogenesis plant cultures.

In a particular embodiment of the method of the invention, to promote *in vitro* plant cell reprogramming towards somatic embryogenesis in a solid medium, the plant hormone concentration is from 0.3 to 2 mg/L, preferably is from 0.3 to 1.5 mg/L. Preferably, the plant hormone concentration is 0.4 mg/L, 0.5 mg/L, 0.6 mg/L, 0.7 mg/L, 0.8 mg/L, 0.9 mg/L or 1 mg/L. More preferably, the plant hormone concentration is 0.5 mg/L.

As a person skilled in the art knows, the induction medium, can further comprises other compounds. Thus, in a particular embodiment, the induction medium further comprises Sommer macronutrients, MS micronutrients and vitamins. In another particular embodiment the induction medium further comprises glutamine and sucrose.

As it has been described above, inventors of the invention have applied the PDE inhibitor/s of the invention in *in vitro* cultures at different concentrations, enhancing proliferation and plant cell reprogramming towards plant embryogenesis or microcallus formation.

Thus, a particular embodiment provides the method of the invention wherein the inhibitor of the invention concentration is from 0.10 to 100 µM.

It is routine practice for a person skilled in the art that the inhibitor concentrations required for promoting plant cell reprogramming towards plant embryogenesis or microcallus formation depend on the culture material and method used. For instance, solid media involve less diffusion and availability of compounds to cells in comparison with liquid media. For this reason, in some embodiments, inhibitor concentrations are lower when used in liquid media than in solid media.

Thus, in a preferred embodiment of the method of the invention, when the isolated plant material is cultured in a liquid medium, the inhibitor concentration is from 0.10 to 19 µM.

In a preferred embodiment of the method of the invention, when the isolated plant material is cultured in a liquid medium and promotes *in vitro* plant cell reprogramming towards plant embryogenesis, the inhibitor of the invention concentration is from 0.10 to 2.50 µM, preferably is from 0.25 to 2 µM. In a more preferred embodiment, the inhibitor concentration is selected from the list consisting of: 0.20 µM, 0.25 µM, 0.30 µM, 0.35 µM, 0.40 µM, 0.45 µM, 0.50 µM, 0.55 µM, 0.60 µM, 0.65 µM, 0.70 µM, 0.75 µM, 0.80 µM, 0.85 µM, 0.90 µM, 0.95 µM, 1 µM, 1.05 µM, 1.10 µM, 1.15 µM, 1.20 µM, 1.25 µM, 1.30 µM, 1.35 µM, 1.40 µM, 1.45 µM, 1.50 µM, 1.55 µM, 1.60 µM, 1.65 µM, 1.70 µM, 1.75 µM, 1.80 µM, 1.85 µM, 1.90 µM, 1.95 µM, and 2 µM. Preferably, the inhibitor concentration is 0.5 µM or 1 µM.

In a particular embodiment of the method of the invention, when the PDE inhibitor is 3-(cyclopropylmethoxy)-N-(3,5-dichloropyridin-4-yl)-4-(difluoromethoxy)benzamide (FDA4.21) and/or 4-(3-cyclopentyloxy-4-methoxyphenyl)pyrrolidin-2-one (FDA4.22), and the isolated plant material is cultured in a liquid medium promoting *in vitro* plant cell reprogramming towards plant embryogenesis, preferably, the inhibitor concentration is 0.50 µM or 1 µM, more preferably 1 µM.

In another particular embodiment of the method of the invention, when the PDE inhibitor is 3-(2,6-Difluorophenyl)-2-methylthio-4-oxo-3,4-dihydroquinazoline (TC2.43) and/or 2-(2-Bromophenyl)-4,5-bis(4-methoxyphenyl)-1H-imidazole (AGF4.17), and the isolated plant material is cultured in a liquid medium promoting *in vitro* plant cell reprogramming towards plant embryogenesis, preferably, the inhibitor concentration is 0.50 µM or 1 µM.

In another particular embodiment of the method of the invention, when the PDE inhibitor is (R)-N-(3-chlorobenzyl)-1-((2-(2-fluorophenyl)-5-methyloxazol-4-yl)methyl)piperidin-3-carboxamide (JHD1.48), and the isolated plant material is cultured in a liquid medium promoting *in vitro* plant cell reprogramming towards plant embryogenesis, preferably, the inhibitor concentration is 0.50 µM.

In another preferred embodiment of the method of the invention, when the inhibitor of the invention is used in liquid media and promotes *in vitro* plant cell reprogramming towards microcallus formation, the inhibitor concentration is from 1 to 19 µM. Preferably, the PDE inhibitor concentration is from 5 to 15 µM. In another more particular embodiment, the PDE inhibitor concentration is 5 µM, 6 µM, 7 µM, 8 µM, 9 µM, 10 µM, 11 µM, 12 µM, 13 µM, 14 µM or 15 µM. More preferably, the PDE inhibitor of the invention concentration is 10 µM.

It is routine practice for a person skilled in the art that the inhibitor concentrations required for promoting plant cell reprogramming towards plant embryogenesis or microcallus formation depend on the culture material and method used. As it has been previously described, solid media involve less diffusion and availability of compounds to cells in comparison with liquid media. For this reason, in some embodiments, inhibitor concentrations are higher when used in solid media than in liquid media.

Thus, in a preferred embodiment of the method of the invention, the inhibitor concentration is from 20 to 80 µM. Preferably, the inhibitor concentration is from 30 to 70 µM, more preferably is from 40 to 60 µM. In a still preferred embodiment, the inhibitor concentration is selected from the list consisting of: 40 µM, 41 µM, 42 µM, 43 µM, 44 µM, 45 µM, 46 µM, 47 µM, 48 µM, 49 µM, 50 µM, 51 µM, 52 µM, 53 µM, 54 µM, 55 µM, 56 µM, 57 µM, 58 µM, 59 µM and 60 µM. More preferably, the inhibitor concentration is 50 µM.

As it has been described above, the inhibitors of the invention have been successfully applied in a forest, a crop and an herbaceous plant, fostering plant cell reprogramming towards plant embryogenesis or microcallus formation. Thus, a particular embodiment provides the method of the invention wherein the plant material belongs to a crop plant, a forest plant or an herbaceous plant.

Preferably, when it promotes *in* vitro plant cell reprogramming towards plant embryogenesis, the plant material belongs to a crop plant or a forest plant.

In a preferred embodiment, the crop plant is selected from the list consisting of: *Hordeum spp., Zea mays, Secale spp, Setaria italica, Panicum miliaceum, Avena spp., Oryza spp., Triticum spp, Sorghum spp., Triticale (Hybrid of Triticum aestivum and Secale cereale), Pennisetum glaucum, Eleusine coracana, Phalaris canariensis, Cynara spp., Daucus carota, Piper spp, Trifolium spp, Brassica spp, Lactuca spp, Mentha spp, Allium spp., Pisum sativum, Capsicum spp, Solamum spp, Cucurbita spp, Chenopodium quinoa, Rubus spp, Spinacia oleracea, Beta spp, Solanum spp., Helianthus spp., Gossypium spp, Arachis hypogaea, Cannabis sativa, Saccharum officinarum, Linum spp., Glycine spp., Nicotiana spp, Medicago spp.* and *Agrostis spp.*

More preferably, the crop plant belongs to the *Brassica spp.,* even more preferably to *Brassica napus sp.*

In another preferred embodiment, the forest plant is selected from the list consisting of: *Araucaria spp., Cupressus spp, Juniperus spp., Abies spp., Cedrus spp, Larix spp, Picea spp., Pinus spp., Pseudotsuga spp, Taxus spp., Ginkgo biloba, Acer spp., Anacardium occidentale, Mangifera spp, Pistacia spp, Cocos nucifera, Phoenix spp, Betula spp., Corylus spp, Carica papaya, Hevea brasiliensis, Acacia spp., Robinia spp, Castanea spp, Fagus spp, Quercus spp., Cinnamomum spp., Laurus spp, Persea spp., Morus spp., Psidium spp., Fraxinus spp., Olea europaea, Platanus spp, Malus spp., Prunus spp., Pyrus spp., Coffea spp., Citrus spp., Populus spp., Salix spp., Theobroma cacao, Camellia spp., Ulmus spp., Tamarillo, (Cyphomandra betacea (Cav.) (Sendtn.)* and *Eucalyptus spp.*

More preferably, the forest plant belongs to the *Quercus spp.,* even more preferably to *Quercus suber sp.*

In a particular embodiment of the method of the invention, when it promotes *in* vitro plant cell reprogramming towards microcallus formation, the plant material belongs to an herbaceous plant.

In a preferred embodiment, the herbaceous plant is selected from the list consisting of: *Arabidopsis spp., Campanula latifolia spp., Geranium psilostemon spp., Iris latifolia spp., Rodgersia aesculifolia spp., Artemisia ludoviciana 'Silver King' spp., Kniphofia hybrids spp., Lychnis coronaria spp., Stachys byzantina spp., Alchemilla mollis spp., Astilbe x arendsii spp., Hakonechloa macra 'Aureola' spp., Nepeta x faassenii spp., Allium christophii spp., Campanula persicifolia spp., Goniolimon tataricum spp., Tanacetum parthenium 'Aureum' spp., Allium cernuum spp., Adiantum venustum spp., Deschampsia cespitosa spp., Ophiopogon planiscapus spp., Athyrium filix-femina spp., Kirengeshoma palmata spp., Equisetum hyemale spp., Thymus serpyllum spp., Agastache foeniculum spp., Erigeron karvinskianus spp., Oenothera biennis spp., Bidens aurea spp., Helianthus annuus spp., Bergenia crassifolia spp., Iberis umbellata spp., Liriope muscari spp., Pontederia cordata spp., Asclepias incarnates spp., Helianthemum nummularium spp.,* and *Rhodiola rosea spp.*

Preferably, the herbaceous plant belongs to *Arabidopsis spp.,* more preferably to *Arabidopsis thaliana sp.*

The terms used to define the method of the invention have been described in the previous inventive aspect, and apply equally, as well as their particular embodiments, to the method of the invention.

### DESCRIPTION OF THE DRAWINGS

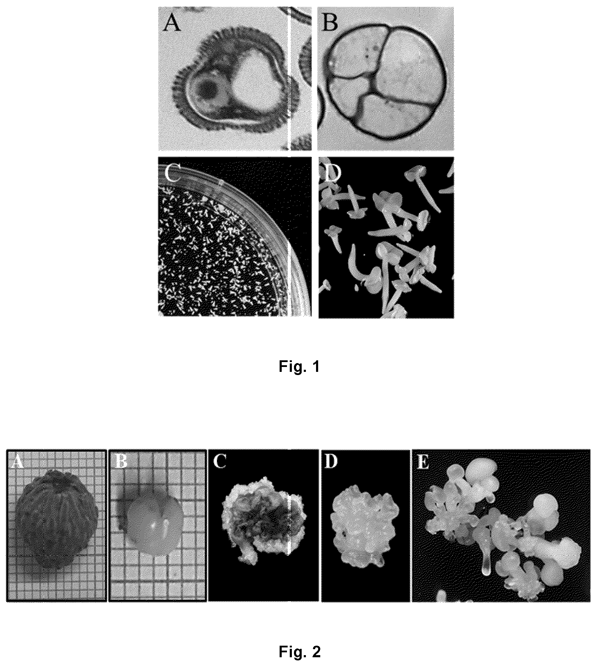

**Fig. 1****.** *In vitro* microspore embryogenesis of *B*. *napus.* **(A, B)** representative micrographs of toluidine stained sections of isolated vacuolated microspore (culture initiation) **(A)** and proembryo (first morphological sign of embryogenesis initiation) **(B). (C,** D) Cotyledonary embryos developed from isolated microspore culture in liquid medium, panoramic view of a region of the culture plate (C) and detail at higher magnification (D).
**Fig. 2****.** Somatic embryogenesis in *Q*. *suber.* **(A)** Acorn. **(B)** Immature zygotic embryo, before induction. (C) Embryogenic masses emerging from the explant after induction. (D) Embryogenic mass in proliferation. **(E)** Embryos at different developmental stages, emerging from embryogenic masses and other embryos, some of them have differentiated fully mature cotyledonary embryos.
**Fig. 3****.** Proliferating protoplasts from mesophyll tissue of Arabidopsis seedlings. Overlay images from bright field and epiflourescence recordings of protoplasts from an H2B-YFP overexpressing Arabidopsis line, recorded after immobilization for the days indicated. The nuclear localized YFP fluorescence (white signal, arrows) indicates the position and number of nuclei.
**Fig. 4****.** Effects of small molecules FDA4.21 and FDA4.22 (PDE4 inhibitors) over embryogenesis induction efficiency in *B*. *napus* microspore cultures. Columns indicate percent change of proembryos at 4 days and refer to the mean percentage of proembryos in control cultures ± SEM (standard error of the mean) which has been normalized to 100%. Different letters indicate significant differences between control and treated cultures according to ANOVA and Tukey tests at P < 0.05.
**Fig. 5****.** Effects of small molecule TC2.43 (PDE7 inhibitor) over embryogenesis induction efficiency in *B*. *napus* microspore cultures. Columns indicate percent change of proembryos at 4 days and refer to the mean percentage of proembryos in control cultures ± SEM (standard error of the mean) which has been normalized to 100%. Different letters indicate significant differences between control and treated cultures according to ANOVA and Tukey tests at P < 0.05.

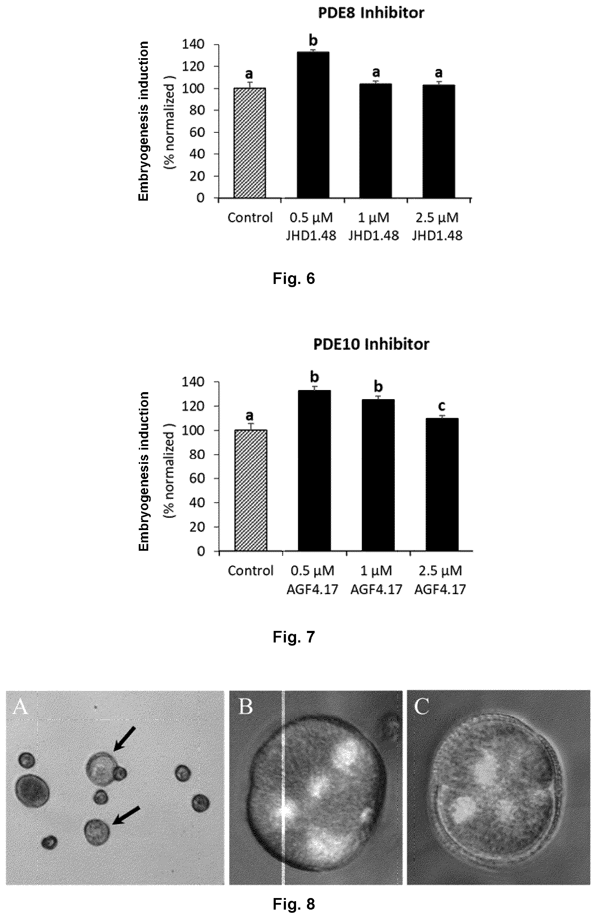

**Fig. 6****.** Effects of small molecule JHD1.48 (PDE8 inhibitor) over embryogenesis induction efficiency in *B*. *napus* microspore cultures. Columns indicate percent change of proembryos at 4 days and refer to the mean percentage of proembryos in control cultures ± SEM (standard error of the mean) which has been normalized to 100%. Different letters indicate significant differences between control and treated cultures according to ANOVA and Tukey tests at P < 0.05.
**Fig. 7****.** Effects of small molecule AGF4.17 (PDE10 inhibitor) over embryogenesis induction efficiency in *B*. *napus* microspore cultures. Columns indicate percent change of proembryos at 4 days and refer to the mean percentage of proembryos in control cultures ± SEM (standard error of the mean) which has been normalized to 100%. Different letters indicate significant differences between control and treated cultures according to ANOVA and Tukey tests at P < 0.05.
**Fig. 8****.** Proembryos in treated and untreated cultures of microspore embryogenesis of B. *napus.* **(A)** Culture after 4 days, where proembryos (arrows) coexist with non-responding and dead microspores (smaller structures). **(B, C)** DAPI staining reveals that proembryos from untreated (B) and treated (C) cultures contain several nuclei (white signals) into the proembryos, indicating embryogenesis initiation.

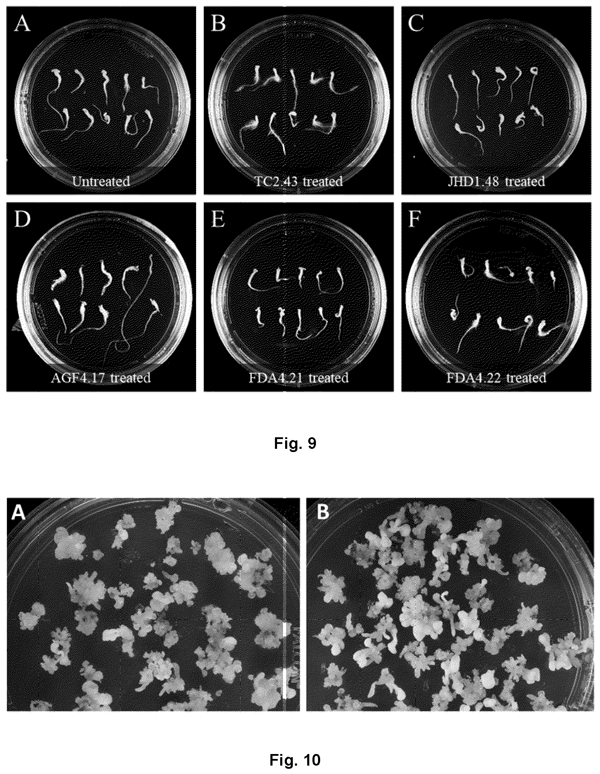

**Fig. 9****.** Germination capacity of embryos produced in control and treated microspore cultures of B. *napus.* Germinating embryos from untreated **(A)** and treated cultures with the inhibitors TC2.43 **(B),** JHD1.48, (C), AGF4.17 **(D),** FDA4.21 **(E)** and FDA4.22 **(F),** all of them showing well-developed roots and hypocotyls in most embryos.
**Fig. 10****.** Effects of PDE8 inhibitor on somatic embryogenesis of *Quercus suber.* **(A)** Untreated culture. **(B)** Culture treated with PDE8 inhibitor JHD1.48.
**Fig. 11****.** Quantification of the effects of PDE inhibitors over somatic embryo production in *Q*. *suber.* (A) PDE7 inhibitor, (B) PDE8 inhibitor, (C) PDE4 inhibitors. Columns indicate mean number of embryos produced after 30 days per gram of embryogenic mass at culture initiation ± SEM (standard error of the mean), referred to the mean number of embryos in control cultures which has been normalized to 100. Grey columns: control cultures, black columns: cultures treated with the inhibitors.
**Fig. 12****.** Microwell overview of PDE7 inhibitor-treated mesophyll protoplasts. Exemplary whole microwells of a 96-well plate with microcalli formed from immobilized Arabidopsis mesophyll protoplasts, pulse-treated with 10 µM 3-(2,6-Difluorophenyl)-2-methylthio-4-oxo-3,4-dihydroquinazoline (TC2.43) (PDE7 inhibitor, right) or an equivalent volume of DMSO (left) for 48 h. Cells were further incubated in proliferation medium and images were recorded 10 days after immobilization. Microcalli are identified by their YFP labelled nuclei and larger size (arrows).
**Fig. 13****.** Effects of small molecule TC2.43 (PDE7 inhibitor) on proliferation efficiency in isolated Arabidopsis mesophyll protoplasts. Immobilized protoplasts were pulse-treated with compounds indicated in proliferation medium for 48 h. Columns indicate percent change of microcallus formation after 7 days cultivation and refer to the mean percentage of microcalli in control cultures ± SEM (standard error of the mean) which has been normalized to 100%. Different numbers of asterisks indicate significant differences between control and samples treated with small molecules according to Student's t-tests with ***, P < 0.001, *, P < 0.05.

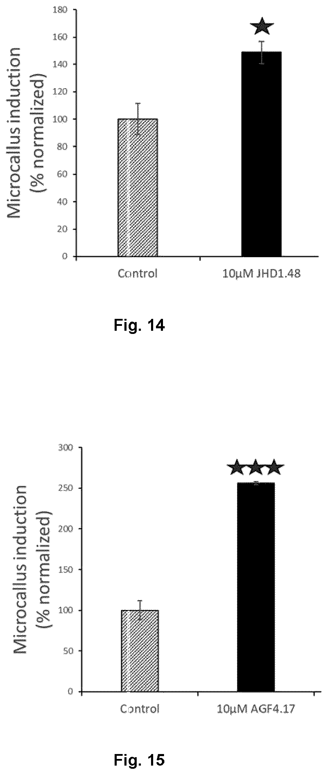

**Fig. 14****.** Effects of small molecule JHD1.48 (PDE8 inhibitor) on proliferation efficiency in isolated Arabidopsis mesophyll protoplasts. Immobilized protoplasts were pulse-treated with compounds indicated in proliferation medium for 48 h. Columns indicate percent change of microcallus formation after 7 days cultivation and refer to the mean percentage of microcalli in control cultures ± SEM (standard error of the mean) which has been normalized to 100%. Asterisks indicate significant differences between control and samples treated with small molecules according to Student's t-tests with *, P < 0.05.
**Fig. 15****.** Effects of small molecule AGF4.17 (PDE10 inhibitor) on proliferation efficiency in isolated Arabidopsis mesophyll protoplasts. Immobilized protoplasts were pulse-treated with compounds indicated in proliferation medium for 48 h. Columns indicate percent change of microcallus formation after 7 days cultivation and refer to the mean percentage of microcalli in control cultures ± SEM (standard error of the mean) which has been normalized to 100%. Asterisks indicate significant differences between control and samples treated with small molecules according to Student's t-tests with ***, P < 0.001.

### Examples

### 1. METHODS

### 1.1. PDE inhibitors on in vitro plant cultures to promote plant cell reprogramming towards plant embryogenesis or microcallus formation.

Five small molecules that belong to an in-house chemical library and have shown inhibitory effects over different types of mammalian PDEs were used.

### PDE inhibitors:

- 3-(cyclopropylmethoxy)-N-(3,5-dichloropyridin-4-yl)-4-(difluoromethoxy)benzamide (FDA4.21)
- 4-(3-cyclopentyloxy-4-methoxyphenyl)pyrrolidin-2-one (FDA4.22). FDA4.21 (Roflumilast) and FDA4.22 (Rolipram) are commercial inhibitors of mammalian PDE4.
- 3-(2,6-Difluorophenyl)-2-methylthio-4-oxo-3,4-dihydroquinazoline (TC2.43). Inhibitor of mammalian PDE7 (Castaño et al., ChemMedChem., 4(5):866-76 (2009)).
- (R)-N-(3-chlorobenzyl)-1-((2-(2-fluorophenyl)-5-methyloxazol-4-yl)methyl)piperidin-3-carboxamide (JHD1.48). Inhibitor of mammalian PDE8 (ES2696516A1).
- 2-(2-Bromophenyl)-4,5-bis(4-methoxyphenyl)-1H-imidazole (AGF4.17): Inhibitor of mammalian PDE10 (Garcia et al., Future Med Chem., 9(8):731-748 (2017)).

The molecular structure of the PDE inhibitors is shown here:

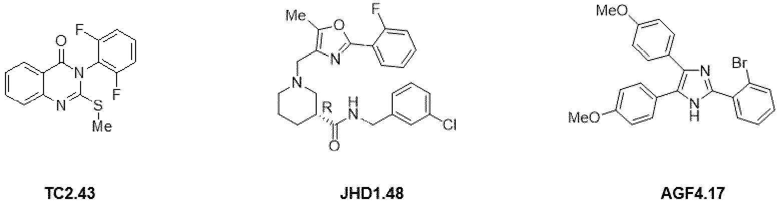

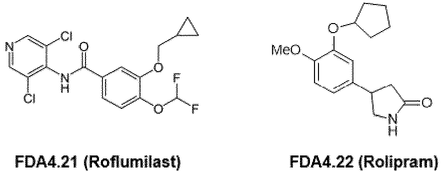

First assays were performed with all the inhibitors in *Brassica napus* microspore cultures in liquid media, where 3 different concentrations (0.5µM, 1.0µM and 2.5µM) were tested for each of inhibitor.

After evaluation of the effects of the inhibitors on embryogenesis initiation efficiency in *B. napus* microspore embryogenesis, the compounds were tested in the *in vitro* embryogenesis system of *Q*. *suber* (cork oak) at higher concentration (50µM), because it was developed in solid-gelified media.

Moreover, some of the inhibitors, particularly TC2.43, JHD1.48 and AGF4.17, were performed at a concentration of 10 µM in *Arabidopsis thaliana* isolated protoplasts to test their capability to increase the microcallus formation.

The inhibitors were added from culture initiation and their effect on embryogenesis or microcallus formation efficiency was assessed. Several plates of the same cultures were kept without the inhibitors, as controls (untreated cultures, i.e. DMSO solution without any PDE inhibitor).

### 1.2. Microspore embryogenesis of Brassica napus, through isolated microspore culture, without inhibitors.

*Brassica napus* L. (rapeseed) cv. 'Topas' line DH407 plants were used as donor plants. Rapeseed seeds were germinated and grew under controlled conditions (relative humidity 60%, 15°C under long-day photoperiod 16 h light and 8 h dark at 10°C) in a growth chamber (Sanyo MLR-351-H) in pots containing a mixture of organic substrate and vermiculite (2/1, v/v). Flower buds containing vacuolated microspores (Fig. 1A), the most responsive stage for microspore induction, were isolated for microspore culture as previously described (Prem et al., 2012 BMC Plant Biology 12, 127).

The selected buds were surface-sterilized in 5.0% (v/v) commercial bleach (5% active chlorine) for 20 min and then rinsed 6-7 times with sterile distilled water. Ten to 15 buds were crushed using a cold mortar and pestle in 5 mL of cold NLN-13 medium (Lichter, R., 1982, Zeitschrift Fur Pflanzenphysiologie 105(5), 427-434) containing 13% sucrose (w/v). The suspension was filtered through 48 µm nylon mesh and the filtrate collected in 15-mL falcon centrifuge tubes. The crushed buds were rinsed with 5 mL NLN-13 to make up the volume to 10 mL and the filtrate was then centrifuged at 1100 rpm for 5 min at 4°C. The pellet was re-suspended in 10 mL of cold NLN-13 and centrifuged as mentioned above. This process was repeated three times for washing of the microspores. The final pellet was suspended in the NLN-13, and the cell density was adjusted to 10,000 cells per mL. The cell suspension was then poured into 90-mm Petri dishes (10 mL per Petri dish) and cultured in darkness.

For embryogenesis induction, microspore cultures were subjected to an *in vitro* stress treatment of 32°C for several days (around 15 days). In response to the inductive treatment, responsive microspores divide and produce multicellular structures or proembryos (Fig. 1B), still confined within the microspore wall (exine). Such structures are considered to be the first sign of embryogenesis initiation; they can be found after 4-6 days in culture. When globular/ heart shaped embryos were observed (around 20 days), cultures were shifted to 25°C on a gyratory shaker at 60 rpm until complete development and maturation of the embryos was observed, normally around 30 days in culture (Fig. 1C).

### 1.3. Somatic embryogenesis of Quercus suber, through immature zygotic embryos culture, without inhibitors.

Immature pollinated acorns were collected from *Quercus suber* L. (cork oak) trees in the countryside (EI Pardo region, Madrid, Spain) during fruit development period (late August and September), transferred to the laboratory and kept at 4°C for one week before *in vitro* culture initiation. Immature acorns (Fig. 2A) were selected at the most responsive stage to somatic embryogenesis induction; they are those with small size, around 1 cm diameter; they contain immature zygotic embryos at the early cotyledonary stage. Immature zygotic embryos (Fig. 2B) were carefully excised from the acorns by dissecting the surrounding tissues with the help of scalpel and forceps. After dissection, explants (immature zygotic embryos) were sterilized by immersion in 70% ethanol for 30 s and in 2% sodium hypochlorite for 20 min, followed by three rinses in sterile distilled water of 10 min each.

Somatic embryogenesis was induced as previously described (Testillano et al. 2018, Plant Cell Culture Protocols, eds. V.M. Loyola-Vargas & N. Ochoa-Alejo. Springer and Bussines Media. pp. 247-256). Explants were first cultured in solid induction medium (Bueno et al., 1992, Physiologia Plantarum 85, 30-34; Manzanera et al., 1993, Silvae Genetics 42, 90-93), which contains Sommer macronutrients, MS micronutrients and vitamins, 0.5mg/L Glutamine, 30g/L Sucrose, and 0.5mg/L Dichlorophenoxyacetic acid (2,4-D), for one month at 25°C and 16/8h light/darkness. During this induction period, cell reprogramming occurred in some responsive cells which initiated the embryogenesis pathway, producing small proembryogenic masses that emerge from the surface (Fig. 2C).

Then, the explants were transferred to solid proliferation medium, with the same composition but growth regulator-free (without 2,4-D). During the next weeks of culture in the proliferation medium, proembryogenic masses (Fig. 2D) proliferated; they produce new embryogenic masses and embryos, which in turn give rise to new embryos, that developed to fully developed cotyledonary embryos, by recurrent and secondary embryogenesis (Fig. 2E).

### 1.4. Treatment with PDE inhibitors on microspore embryogenesis cultures of B. napus in liquid media

The compounds were added to the microspore liquid culture media by using stock solutions of 10 mM in dimethyl sulfoxide (DMSO). Appropriate volumes of stock solutions of the compounds were added to the culture media to get the selected working concentrations of the inhibitors, keeping DMSO concentration below 0.2%. Concentrations of 0.5µM, 1.0µM and 2.5µM were tested for each of inhibitor.

### 1.5. Evaluation of the effect of PDE inhibitors over in vitro embryogenesis induction efficiency in isolated microspore cultures of B. napus

Embryogenesis induction efficiency was quantified in control (untreated) and treated-cultures by the number of proembryos formed (considered the first sign of embryogenesis initiation), as previously described (Berenguer et al., 2017, Front Plant Sci 8, 1161). Proembryos were easily identified under inverted microscope in 4 day-culture plates as rounded multicellular structures with higher size and density than microspores, still surrounded by the exine (special microspore wall). Randomly obtained micrographs from inverted microscope were collected from untreated and treated microspore culture plates. Mean percentage of proembryos per plate were obtained from three independent experiments per treatment. A minimum of 1000 proembryos were counted for each treatment. Results on proembryos were expressed as percentages (percent change) and referred to the mean percentage of proembryos in control cultures, which has been normalized to 100%.

In order to evaluate whether proembryo structures of treated cultures, identified under the inverted microscope for quantification, were actually dividing microspores, similar to the same structures from control cultures, a simply staining technique was performed to visualize nuclei inside proembryos. Samples from control and treated-cultures of 4 days at 32°C, containing proembryos, were stained with 10 µg/mL 4',6-diamidine-2-phenyl indole dihydrochloride (DAPI) as previously described (Solís et al., 2008, Plant Science 174(6), 597-605). Squash preparations were analysed under fluorescence microscopy using UV excitation for observing nuclei.

### 1.6. Evaluation of quality/germination capacity of embryos produced after treatment with PDE inhibitors

To evaluate the quality of embryos produced in microspore embryogenesis cultures in the presence of the inhibitors, embryo germination assays were performed. *Brassica napus* microspore cotyledonary embryos originated from control and treated-cultures were used for *in vitro* embryo germination and conversion to plantlets as previously described (Prem et al., 2012 BMC Plant Biology 12, 127). The 34 - 40 old dicotyledonous embryos, after air desiccation on sterile filter paper were germinated in MS (Murashige and Skoog) medium containing sucrose 2 % (w/v) and gelled with 7 g/L bacteriological agar (w/v). Microspore derived-embryos were incubated for 15 - 20 days at 18°C in darkness conditions till activation of radicle and plumule, and quantified in terms of percentage of embryos showing normal growth, similar to zygotic embryo germination.

### 1.7. Treatments with PDE inhibitors on somatic embryogenesis cultures of Q. suber in solid media

Since the *in vitro* system of *Q. suber* was two-step process in solid culture media, a different strategy than in liquid microspore cultures was applied for the treatments with the inhibitors. During *in vitro* embryogenesis of *Q. suber,* after incubation in induction medium, the transfer of explants to proliferating medium involves the multiplication of proembryogenic masses, embryogenesis initiation, by recurrent and secondary embryogenesis, and embryo development. Therefore, treatments with the inhibitors were performed during the first 15 days at 25°C in proliferating media, and afterwards, explants with emerging embryos were transferred to fresh proliferating media without the inhibitor.

Since solid media involve much less diffusion and availability of compounds to cells in comparison with liquid media, as referred in other *in vitro* systems, the concentration of the inhibitors used in solid media was around 20X higher than in liquid media, particularly 50 µM. Appropriate volumes of stock solutions of 10 mM in DMSO of the selected compounds were added to cooled media, before its gelling, keeping DMSO concentration below 0.2%. Mock parallel plates of the same cultures were kept as controls.

### 1.8. Evaluation of the effect of PDE inhibitors over somatic embryogenesis efficiency in Q. suber

Embryogenesis induction efficiency was quantified in control and treated-cultures by the number of embryos (torpedo to cotyledonary stage) produced by 15 days of treatment (culture medium containing the inhibitor) followed by 30 days of recovery (culture medium without inhibitor). Embryo production was estimated as the number of embryos originated per gram of embryogenic masses at culture initiation.

### 1. 9. Plant cultivation and protoplast isolation of Arabidopsis

Seeds of an *Arabidopsis thaliana* line overexpressing histone 2B fused with yellow flourescent protein (H2B-YFP) were surface-sterilized and sown on MS agar. After 24 h stratification, plates were cultured vertically for 7 days under long day conditions (16 h light - 8 h dark) at 22°C. Prior to seedling harvest, plates were kept vertically in the dark at 4°C for 24 h. Protoplasts were isolated from hypocotyl and cotyledons including primary leaves according to Dovzhenko et al., Protoplasma, 222(1-2):107-11 (2003).

### 1.10. Protoplast immobilization and image acquisition

Protoplast density was determined by cell counting in a Fuchs-Rosenthal chamber. For immobilization, protoplasts were mixed with equal volumes of 1.2 % (w/v) low melting agarose in protoplast buffer, prewarmed at 40°C and immediately pipetted into a 96-well plate (Ibidi, Germany), prewarmed at 34 °C with 125 µL per well. The plate was immediately centrifuged (2 min, 30g) and incubated at 4°C for 5 min, 200 µL of cultivation medium was added on top of each well and the protoplasts were cultivated at 22°C in the dark. Cultivation medium was composed of macro and micro elements based on Kao KN and Michayluk MR. Planta., 126(2):105-10 (1975), supplemented with 2 µM of 2,4-D and 1 µM of thidiazurone. For PDE inhibitors treatments, PDE inhibitors were dissolved in DMSO and added to the medium with the concentration given (liquid media). After 48 h and at 22 °C, the cultivation medium was removed and replaced with fresh medium after two washing steps performed with cultivation medium.

An automated microscope (MORE1, Till I.D. GmbH, Munich, Germany) was used for the analysis of protoplast development and for the detection of YFP fluorescence. Transillumination was recorded with 10x0.45 and 20x0.8 objectives (Zeiss); epifluorescence was recorded after excitation with single-mode diode lasers (iBeam smart, Toptica) with excitation of 550 nm (YFP)/green emission filter. Image acquisition was performed using the SIAM software (Till I.D. GmbH, Munich, Germany).

### 1.11. Image processing and data analysis

Images were recorded immediately after immobilization and 10 days after immobilization (DAls). Image processing was performed with Fiji an image processing package distributed by ImageJ2 (Schindelin J, et al., Nat Methods, 9(7):676-82 (2012); Rueden, C.T et al., BMC Bioinformatics, 18, 529 (2017)). Raw tile image stacks were reconstituted to full-well images using the stitching plugin (Preibisch et al., Bioinformatics, 25, 1463-1465 (2009)). Image segmentation was performed with U-net (Falk et al., Nat. Methods, 16, 67-70 (2018)). Quantification of proliferating cells was performed by exploiting shape differences of dividing or closely adjacent nuclei after the application of EDM binary operations on epifluorescence images in comparison with circular fluorescence signals from non-dividing mononucleic cells (Brocher Int. J. Image Process, 8, 30-48 (2014)). Corresponding cellular shape parameters from proliferating microcalli were determined from selecting segmented transillumination images after filtering with processed epiflourescence images using the BioVoxxel binary feature extraction function. An exemplary image series of protoplasts from proliferating mesophyll in cultivation medium is shown in Fig. 3.

### 2. RESULTS

### 2.1. Effect of PDE inhibitors over microspore embryogenesis induction efficiency in Brassica napus

The efficiency of embryogenesis induction was evaluated in microspore control cultures and cultures treated with the small molecule inhibitors at different concentrations. The presence of the inhibitors in the culture media affected the production of proembryos (first sign of embryogenesis initiation) in comparison with control cultures, being the proportion of proembryos different depending on the concentration used.

Inhibitors were used at 3 concentrations: 0.5µM, 1.0 µM and 2.5µM. Quantification of proembryos produced, as first sign of embryogenesis initiation, in control and treated cultures showed that the two PDE4 inhibitors tested led to a significant increase of the production of proembryos, at 0.5 and 1.0 depending on the inhibitor, being the increase of embryogenesis initiation in the range of 32-47%, compared to control (Fig. 4).

Regarding the assays with PDE7, PDE8 and PDE10 inhibitors, the results showed significant increases in microspore embryogenesis induction efficiency in the range of 32-35%, in cultures treated with the three inhibitors. (Figs. 5, 6 and 7).

To confirm that proembryos quantified in treated cultures were multicellular microspores that have initiated embryogenesis, squash preparations from control and treated cultures at 4 days were stained with DAPI and observed under fluorescence microscopy. Results showed that proembryos from treated cultures contained several nuclei, as in control cultures, indicating that they were actually dividing microspores that likely initiated embryogenesis (Fig. 8).

The quality of the embryos produced in microspore cultures treated with the inhibitors was evaluated by germination assays. Fully developed cotyledonary embryos from control and treated 30-40 days cultures were desiccated and cultured under germination conditions. Results showed that embryos from treated cultures germinated very well, producing roots and hypocotyl, similarly and in the same proportion than embryos from control cultures (Fig. 9).

### 2.2. Effect of PDE inhibitors over somatic embryogenesis cultures of Quercus suber

In order to evaluate the possibility to extend the findings from B. napus to more distant species and processes, the inhibitors of PDE4, PDE7 and PDE8 were applied to a forest woody species Q. *suber.* PDE inhibitors were applied to the *in vitro* system of somatic embryogenesis from immature zygotic embryos, a two-step culture in solid media.

Inhibitor treatments were applied on embryogenic masses at concentration around 20X higher than in liquid media, particularly 50 µM, because of the lower diffusion and availability of compounds in gelled medium. The effects of the compounds over embryogenesis efficiency were assessed by the quantification of the embryos produced after 15 days of treatment followed by 30 days in fresh medium.

Control (untreated) cultures developed a number of somatic embryos (Fig. 10A, white structures), while cultures treated with the PDE8 inhibitor showed higher number of somatic embryos (Fig. 10B).

Quantification of the number of embryos per gram of embryogenic masses at the beginning of culture showed that treatments with the three types of inhibitors increased embryogenesis induction efficiency and lead to higher embryo production, being the increment of 42-36% for inhibitors of PDE7 and PDE8 (Figs. 11A, 11B), and around 20% for inhibitors of PDE4 (Fig. 11C).

### 2.3. Effect of PDE inhibitors over mesophyll protoplast microcallus induction efficiency in Arabidopsis thaliana

PDE inhibitors were tested for their capability to increase the proliferation rate of Arabidopsis protoplasts isolated from leaves. For this, immobilized protoplasts were overlaid with cultivation medium supplemented with inhibitors in concentrations from 1 to 10 µM for 48 h, inhibitors were removed by washing and cultivation medium was added and cultivation continued. Microscopic images were recorded 10 days after immobilization and proliferating microcalli were identified by the presence of multiple fluorescing nuclei visualized by exploiting the nuclear-encoded H2B-YFP marker (see Fig. 3). As mock control, protoplasts were treated with the equivalent volume of DMSO. This revealed for the small molecule inhibitor of PDE7 a visibly increased number of microcalli formed (see Fig. 12).

Proliferation rates were quantified and normalized to the number of protoplasts immobilized in each well. This revealed an increased rate of microcalli formation of 140% for 1 µM TC2.43 while 10 µM TC2.43 increased the rate by 200% (Fig. 13), each relative to the untreated control.

Similarly, if the small molecule JHD1.48, which inhibits mammalian PDE8 was applied with 10 µM concentration to mesophyll protoplasts for 48 h, microcalli formation rate was increased by 50% (Fig. 14).

Moreover, application of AGF4.17 showed the strongest induction of microcallus formation among the inhibitors tested with 250% compared with the untreated control. (Fig. 15).

### 3. CONCLUSIONS

The PDE inhibitors described before have demonstrated capacity to increase plant cell reprogramming, embryogenesis induction and embryo production yield in two different species, a crop and a forest plant, as well as plant cell reprogramming and proliferation in protoplast cultures in Arabidopsis. Moreover, treatments with these inhibitors have been successfully applied to different *in vitro* protocols, in liquid or solid media. These results suggest that the use of these compounds could be extended to promote *in vitro* plant cell reprogramming a proliferation towards plant embryogenesis, either somatic or microspore embryogenesis, or microcallus formation in a wider range of plant species and *in vitro* systems, for further plant regeneration.

## Claims

1. Use of at least one phosphodiesterase (PDE) inhibitor to promote *in vitro* plant cell reprogramming towards plant embryogenesis or microcallus formation, wherein the PDE inhibitor is selected from the list consisting of:
(i) 3-(2,6-Difluorophenyl)-2-methylthio-4-oxo-3,4-dihydroquinazoline,
(ii)(R)-N-(3-chlorobenzyl)-1-((2-(2-fluorophenyl)-5-methyloxazol-4-yl)methyl)piperidin-3-carboxamide,
(iii) 2-(2-Bromophenyl)-4,5-bis(4-methoxyphenyl)-1H-imidazole,
(iv)3-(cyclopropylmethoxy)-N-(3,5-dichloropyridin-4-yl)-4-(difluoromethoxy)benzamide,
(v)4-(3-cyclopentyloxy-4-methoxyphenyl)pyrrolidin-2-one
and any combination thereof.

2. The use according to claim 1, wherein the plant embryogenesis is microspore embryogenesis or somatic embryogenesis.

3. The use according to claim 1 or 2, wherein the PDE inhibitor concentration is from 0.10 to 100 µM.

4. The use according to any one of claims 1 to 3, wherein the plant is a crop plant, preferably *Brassica spp.,* more preferably *Brassica napus sp.*; wherein the plant is a forest plant, preferably *Quercus spp.,* more preferably *Quercus suber sp*; or wherein the plant is an herbaceous plant, preferably *Arabidopsis spp.,* more preferably to *Arabidopsis thaliana sp.*

5. A method of promoting *in vitro* plant cell reprogramming towards plant embryogenesis or microcallus formation, comprising:
a) Culturing an isolated plant material with at least one PDE inhibitor,
wherein the PDE inhibitor is selected from the list consisting of:
(i) 3-(2,6-Difluorophenyl)-2-methylthio-4-oxo-3,4-dihydroquinazoline,
(ii)(R)-N-(3-chlorobenzyl)-1-((2-(2-fluorophenyl)-5-methyloxazol-4-yl)methyl)piperidin-3-carboxamide,
(iii) 2-(2-Bromophenyl)-4,5-bis(4-methoxyphenyl)-1H-imidazole,
(iv)3-(cyclopropylmethoxy)-N-(3,5-dichloropyridin-4-yl)-4-(difluoromethoxy)benzamide,
(v) 4-(3-cyclopentyloxy-4-methoxyphenyl)pyrrolidin-2-one
and any combination thereof.

6. The method according to claim 5, wherein the plant embryogenesis is microspore embryogenesis or somatic embryogenesis.

7. The method according to claim 5 or 6, wherein the isolated plant material is cultured in a solid medium or in a liquid medium.

8. The method according to any one of claims 5 to 7, wherein the PDE inhibitor concentration is from 0.10 to 100 µM.

9. The method according to claim 8, wherein the PDE inhibitor concentration is from 0.10 to 19 µM when the isolated plant material is cultured in a liquid medium.

10. The method according to claim 8, wherein the PDE inhibitor concentration is from 20 to 80 µM, preferably 50 µM, when the isolated plant material is cultured in a solid medium.

11. The method according to any one of claims 5 to 10, wherein the plant material belongs to a crop plant, preferably *Brassica spp.,* more preferably *Brassica napus sp.;* wherein the plant material belongs to a forest plant, preferably *Quercus spp.,* more preferably *Quercus suber sp;* or wherein the plant material belongs to an herbaceous plant, preferably *Arabidopsis spp.,* more preferably to *Arabidopsis thaliana sp.*

## Patentansprüche

1. Verwendung mindestens eines Phosphodiesterase(PDE)-Inhibitors zur Förderung einer *In-vitro*-Umprogrammierung von Pflanzenzellen in Richtung pflanzlicher Embryogenese oder Mikrokallusbildung, wobei der PDE-Inhibitor aus der Liste ausgewählt ist, die aus Folgendem besteht:
(i) 3-(2,6-Difluorphenyl)-2-methylthio-4-oxo-3,4-dihydrochinazolin,
(ii) (R)-N-(3-Chlorbenzyl)-1-((2-(2-fluorphenyl)-5-methyloxazol-4-yl)methyl)piperidin-3-carboxamid,
(iii) 2-(2-Bromphenyl)-4,5-bis(4-methoxyphenyl)-1H-imidazol,
(iv) 3-(Cyclopropylmethoxy)-N-(3,5-dichlorpyridin-4-yl)-4-(difluormethoxy)benzamid,
(v) 4-(3-Cyclopentyloxy-4-methoxyphenyl)pyrrolidin-2-on
und jeglicher Kombination davon.

2. Verwendung nach Anspruch 1, wobei die pflanzliche Embryogenese eine Mikrosporen-Embryogenese oder eine somatische Embryogenese ist.

3. Verwendung nach Anspruch 1 oder 2, wobei die PDE-Inhibitor-Konzentration zwischen 0,10 und 100 µM liegt.

4. Verwendung nach einem der Ansprüche 1 bis 3, wobei die Pflanze eine Kulturpflanze ist, vorzugsweise *Brassica spp.,* noch bevorzugter *Brassica napus* sp.; wobei die Pflanze eine Waldpflanze ist, vorzugsweise *Quercus spp.,* noch bevorzugter *Quercus suber sp;* oder wobei die Pflanze eine krautige Pflanze ist, vorzugsweise *Arabidopsis spp.,* noch bevorzugter *Arabidopsis thaliana sp.*

5. Verfahren zur Förderung einer *In-vitro*-Umprogrammierung von Pflanzenzellen in Richtung pflanzlicher Embryogenese oder Mikrokallusbildung, das Folgendes umfasst:
a) Kultivieren eines isolierten Pflanzenmaterials mit mindestens einem PDE-Inhibitor, wobei der PDE-Inhibitor aus der Liste ausgewählt ist, die aus Folgendem besteht:
(i) 3-(2,6-Difluorphenyl)-2-methylthio-4-oxo-3,4-dihydrochinazolin,
(ii) (R)-N-(3-Chlorbenzyl)-1-((2-(2-fluorphenyl)-5-methyloxazol-4-yl)methyl)piperidin-3-carboxamid,
(iii) 2-(2-Bromphenyl)-4,5-bis(4-methoxyphenyl)-1H-imidazol,
(iv) 3-(Cyclopropylmethoxy)-N-(3,5-dichlorpyridin-4-yl)-4-(difluormethoxy)benzamid,
(v) 4-(3-Cyclopentyloxy-4-methoxyphenyl)pyrrolidin-2-on
und jeglicher Kombination davon.

6. Verfahren nach Anspruch 5, wobei die pflanzliche Embryogenese eine Mikrosporen-Embryogenese oder eine somatische Embryogenese ist.

7. Verfahren nach Anspruch 5 oder 6, wobei das isolierte Pflanzenmaterial in einem festen Medium oder in einem flüssigen Medium kultiviert wird.

8. Verfahren nach einem der Ansprüche 5 bis 7, wobei die PDE-Inhibitor-Konzentration zwischen 0,10 und 100 µM liegt.

9. Verfahren nach Anspruch 8, wobei die PDE-Inhibitor-Konzentration zwischen 0,10 und 19 µM liegt, wenn das isolierte Pflanzenmaterial in einem flüssigen Medium kultiviert wird.

10. Verfahren nach Anspruch 8, wobei die PDE-Inhibitor-Konzentration 20 bis 80 µM, vorzugsweise 50 µM, beträgt, wenn das isolierte Pflanzenmaterial in einem festen Medium kultiviert wird.

11. Verfahren nach einem der Ansprüche 5 bis 10, wobei das Pflanzenmaterial zu einer Kulturpflanze gehört, vorzugsweise *Brassica spp.,* noch bevorzugter *Brassica napus sp.;* wobei das Pflanzenmaterial zu einer Waldpflanze gehört, vorzugsweise *Quercus spp.,* noch bevorzugter *Quercus suber sp;* oder wobei das Pflanzenmaterial zu einer krautigen Pflanze gehört, vorzugsweise *Arabidopsis spp.,* noch bevorzugter *Arabidopsis thaliana sp.*

## Revendications

1. Utilisation d'au moins un inhibiteur de la phosphodiestérase (PDE) pour promouvoir la reprogrammation *in vitro* des cellules végétales vers l'embryogenèse végétale ou la formation de microcallus, dans laquelle l'inhibiteur de la PDE est choisi dans la liste constituée de :
(i) 3-(2,6-Difluorophényl)-2-méthylthio-4-oxo-3,4-dihydroquinazoline,
(ii) (R)-N-(3-chlorobenzyl)-1-((2-(2-fluorophényl)-5-méthyloxazol-4-yl)méthyl)pipéridine-3-carboxamide,
(iii) 2-(2-Bromophényl)-4,5-bis(4-méthoxyphényl)-1H-imidazole,
(iv) 3-(cyclopropylméthoxy)-N-(3,5-dichloropyridin-4-yl)-4-(difluorométhoxy)benzamide,
(v) 4-(3-cyclopentyloxy-4-méthoxyphényl)pyrrolidin-2-one
et l'une quelconque combinaison de ceux-ci.

2. Utilisation selon la revendication 1, dans laquelle l'embryogenèse végétale est une embryogenèse de microspores ou une embryogenèse somatique.

3. Utilisation selon la revendication 1 ou 2, dans laquelle la concentration de l'inhibiteur de la PDE va de 0,10 à 100 µM.

4. Utilisation selon l'une quelconque des revendications 1 à 3, dans laquelle la plante est une plante cultivée, de préférence *Brassica spp.,* plus préférablement, *Brassica napus sp.* ; dans laquelle la plante est une plante forestière, de préférence *Quercus spp.,* plus préférablement, *Quercus suber sp ;* ou dans laquelle la plante est une plante herbacée, de préférence *Arabidopsis spp.,* plus préférablement à *Arabidopsis thaliana sp.*

5. Procédé de promotion de la reprogrammation *in vitro* des cellules végétales vers l'embryogenèse végétale ou la formation de microcallus, comprenant :
a) Mise en culture d'un matériel végétal isolé avec au moins un inhibiteur de la PDE, dans lequel l'inhibiteur de la PDE est choisi dans la liste constituée de :
(i) 3-(2,6-Difluorophényl)-2-méthylthio-4-oxo-3,4-dihydroquinazoline,
(ii) (R)-N-(3-chlorobenzyl)-1-((2-(2-fluorophényl)-5-méthyloxazol-4-yl)méthyl)pipéridine-3-carboxamide,
(iii) 2-(2-Bromophényl)-4,5-bis(4-méthoxyphényl)-1H-imidazole,
(iv) 3-(cyclopropylméthoxy)-N-(3,5-dichloropyridin-4-yl)-4-(difluorométhoxy)benzamide,
(v) 4-(3-cyclopentyloxy-4-méthoxyphényl)pyrrolidin-2-one
et l'une quelconque combinaison de ceux-ci.

6. Procédé selon la revendication 5, dans lequel l'embryogenèse végétale est une embryogenèse de microspores ou une embryogenèse somatique.

7. Procédé selon la revendication 5 ou 6, dans lequel le matériel végétal isolé est cultivé dans un milieu solide ou dans un milieu liquide.

8. Procédé selon l'une quelconque des revendications 5 à 7, dans lequel la concentration de l'inhibiteur de la PDE va de 0,10 à 100 µM.

9. Procédé selon la revendication 8, dans lequel la concentration de l'inhibiteur de la PDE va de 0,10 à 19 µM lorsque le matériel végétal isolé est cultivé dans un milieu liquide.

10. Procédé selon la revendication 8, dans lequel la concentration de l'inhibiteur de la PDE va de 20 à 80 µM, de préférence 50 µM, lorsque le matériel végétal isolé est cultivé dans un milieu solide.

11. Procédé selon l'une quelconque des revendications 5 à 10, dans lequel le matériel végétal appartient à une plante cultivée, de préférence *Brassica spp.,* plus préférablement, *Brassica napus sp. ;* dans lequel le matériel végétal appartient à une plante forestière, de préférence *Quercus spp.,* plus préférablement, *Quercus suber sp* ; ou dans lequel le matériel végétal appartient à une plante herbacée, de préférence *Arabidopsis spp.,* plus préférablement à *Arabidopsis thaliana sp.*
